# EUROPEAN PATENT SPECIFICATION

(11) **EP 4 259 185 B1**
(45) Date of publication and mention of the grant of the patent: **28.10.2026**
(21) Application number: 21840344.2
(22) Date of filing: 14.12.2021
(51) Int. Cl.: A61K 38/28, A61P 3/10, A61K 9/00

(54) **METHODS OF TREATING DIABETES**
VERFAHREN ZUR BEHANDLUNG VON DIABETES
MÉTHODES DE TRAITEMENT DU DIABÈTE

(30) Priority: 14.12.2020 US 202063125165 P
(43) Date of publication of application: 18.10.2023
(60) Divisional application: 26206408.2
(73) Proprietor: Eli Lilly and Company, Indianapolis, IN 46285 (US)
(72) Inventor: BUE-VALLESKEY, Juliana M., Indianapolis, Indiana 46206-6288 (US); CARR, Molly Corbett, Indianapolis, Indiana 46206-6288 (US); CHANG, Annette Meeok, Indianapolis, Indiana 46206-6288 (US); CHIEN, Yueh-Ling a/k/a/ Jenny Y. CHIEN, Indianapolis, Indiana 46206-6288 (US); CHIGUTSA, Emmanuel, Indianapolis, Indiana 46206-6288 (US); GARHYAN, Parag, Indianapolis, Indiana 46206-6288 (US); HAUPT, Axel Richard Karl-August, Indianapolis, Indiana 46206-6288 (US); ILAG, Liza L., Indianapolis, Indiana 46206-6288 (US); KATZ, Michelle Lynne, Indianapolis, Indiana 46206-6288 (US); KAZDA, Christof Matthias, Indianapolis, Indiana 46206-6288 (US); SCHNECK, Karen Brenda, Indianapolis, Indiana 46206-6288 (US); TANG, Cheng Cai, Indianapolis, Indiana 46206-6288 (US)
(74) Representative: Eli Lilly and Company Limited
(86) International application number: PCT/US2021/063231
(87) International publication number: WO 2022/132709

(56) References cited:
- WO-A1-2016/178905
- WO-A1-2020/074544
- US-A1- 2020 157 169
- MEHTA ROOPA ET AL: "Practical guidance on the initiation, titration, and switching of basal insulins: a narrative review for primary care", ANNALS OF MEDICINE, vol. 53, no. 1, 1 January 2021 (2021-01-01), SE, pages 999 - 1010, XP093281551, ISSN: 0785-3890, DOI: 10.1080/07853890.2021.1925148
- WYSHAM CAROL ET AL: "Insulin Efsitora versus Degludec in Type 2 Diabetes without Previous Insulin Treatment", THE NEW ENGLAND JOURNAL OF MEDICINE, vol. 391, no. 23, 12 December 2024 (2024-12-12), US, pages 2201 - 2211, XP093281550, ISSN: 0028-4793, DOI: 10.1056/NEJMoa2403953
- CLINICALTRIALS.GOV: "A Study of Insulin Efsitora Alfa (LY3209590) Compared to Glargine in Adult Participants With Type 2 Diabetes Who Are Starting Basal Insulin for the First Time (QWINT-1) (QWINT-1)", 30 October 2024 (2024-10-30), XP093281557, Retrieved from the Internet <URL:https://clinicaltrials.gov/study/NCT05662332>
- RICHARD M BERGENSTAL ET AL: "Once-weekly insulin efsitora alfa: Design and rationale for the QWINT phase 3 clinical development programme", DIABETES, OBESITY AND METABOLISM, BLACKWELL SCIENCE, GB, vol. 26, no. 8, 28 April 2024 (2024-04-28), pages 3020 - 3030, XP072664980, ISSN: 1462-8902, DOI: 10.1111/DOM.15604
- CHURCH CHRISTOPHER ET AL: "Pharmacological characterisation of an ultra-long acting once-weekly insulin-Fc fusion with continuous glucose monitoring", EASD VIRTUAL MEETING, 54TH ANNUAL MEETING OF THE EUROPEAN ASSOCIATION FOR THE STUDY OF DIABETES; BERLIN, GERMANY; 1-5 OCTOBER, 2018, EUROPEAN ASSOCIATION FOR THE STUDY OF DIABETES, 3 October 2018 (2018-10-03), XP009533612
- ANONYMOUS: "A Study of LY3209590 in Participants With Type 2 Diabetes Mellitus - Full Text View - ClinicalTrials.gov", 9 November 2018 (2018-11-09), XP055895530, Retrieved from the Internet <URL:https://clinicaltrials.gov/ct2/show/NCT03736785> [retrieved on 20220225]

## Description

The present invention relates to treating diabetes. More particularly, the present invention relates to treating diabetes with insulin efsitora alfa.

Diabetes is a chronic disorder characterized by hyperglycemia resulting from defects in insulin secretion, insulin action, or both. Type 1 diabetes (T1D) is characterized by little or no insulin secretory capacity, and patients with T1D require insulin for survival. Type 2 diabetes (T2D) is characterized by elevated blood glucose levels resulting from impaired insulin secretion, insulin resistance, excessive hepatic glucose output, and/or contributions from all of the above. In many patients with T2D, the disease progresses to a requirement for insulin therapy.

Because T1D patients produce little or no insulin, effective insulin therapy generally involves the use of two types of exogenously administered insulin: a rapid-acting, mealtime or prandial insulin provided by bolus injections, and a long-acting, basal insulin, administered once or twice daily to control blood glucose levels between meals and the overnight period. Treatment of patients with T2D typically begins with prescribed weight loss, exercise, and a diabetic diet, but when these measures fail to control elevated blood sugars, then oral medications and incretin-based therapy may be necessary. When these medications are still insufficient, treatment with insulin is considered. T2D patients whose disease has progressed to the point that insulin therapy is required are generally started on a single daily injection of a long-acting, basal insulin.

Basal insulin analogs currently available include insulin glargine, sold under the tradenames LANTUS^{®}, TOUJEO^{®}, BASALGLAR^{®} and SEMGLEE^{®}, insulin detemir, sold under the tradename LEVEMIR^{®}, and insulin degludec, sold under the tradename TRESIBA^{®}. These insulins are each indicated for once-daily administration.

Treatment regimens involving daily injections of existing insulin therapies can be complicated and painful to administer and can result in undesired side effects, such as hypoglycemia and weight gain. Therefore, even after initiation of insulin therapy, many diabetic patients are unwilling or unable to comply, or are incapable of complying, with the insulin therapy necessary to maintain close control of blood glucose levels. Research is being conducted to identify insulin products with longer duration of action; thus, requiring fewer injections than currently available insulin products, including as infrequently as once-weekly. Such products would have the potential to improve acceptance and compliance.

There are, however, potential problems associated with administration of products with prolonged insulin activity. For example, prolonged insulin activity could theoretically carry a higher risk for hypoglycemic events or a longer duration of such events and/or weight gain. In addition, administration of a product having prolonged insulin activity once-weekly may require multiple weeks to reach a steady state of insulin activity, resulting in sub-optimal control of hyperglycemia in the interim. While increased risk of hypoglycemia, weight gain and/or sub-optimal hyperglycemia control could be potential downsides of products having longer duration of insulin action than existing insulins, such risks could be managed or eliminated through use of optimized doses and dosing regimens.

WO2014/009316 describes insulin derivatives which are stated to have a long enough time of action that it is sufficient to administer them with a frequency of about once weekly in order for the diabetic patient to get a sufficient basal administration of insulin. A treatment regimen for these derivatives is proposed in WO2016/001185.

US2016/0324932 describes fusion proteins having prolonged duration of action at the insulin receptor sufficient for dosing as infrequently as once-weekly, including BIF. A specific dosing regimen is not described.

There remains a need for insulin therapies requiring fewer injections than currently available insulin products. There also remains a need for methods of treatment using such insulin therapies either without increasing or with reducing the risk of hypoglycemia compared to currently available insulin products. There remains a need for insulin therapies requiring fewer injections than currently available insulin products. There remains a need for insulin therapies providing enhanced glycemic control as compared to currently available insulin products. There also remains a need for methods of treatment using such insulin therapies either without increasing or with reducing the risk of weight gain compared to currently available insulin products. There also remains a need for insulin therapies to be administered in a manner that allows patients to quickly reach steady-state serum levels.

### SUMMARY OF THE INVENTION

Accordingly, the present invention provides insulin efsitora alfa for use in the treatment of diabetes, wherein the treatment comprises providing glycemic control by:
a) administering to an insulin naïve subject an initial dose of weekly insulin efsitora alfa that is a 300 U loading dose; and
b) administering to said subject one or more weekly maintenance doses once-weekly starting one week after administration of the initial dose.

### DETAILED DESCRIPTION OF THE INVENTION

Insulin efsitora alfa, also known as BIF, comprises a dimer of an insulin receptor agonist fused to a human IgG Fc region, wherein the insulin receptor agonist comprises an insulin B-chain analog fused to an insulin A-chain analog through the use of a first peptide linker and wherein the C-terminal residue of the insulin A-chain analog is directly fused to the N-terminal residue of a second peptide linker, and the C-terminal residue of the second peptide linker is directly fused to the N-terminal residue of the human IgG Fc region. BIF is identified by CAS registry number 2131038-11-2, which provides the following chemical names: (1) Insulin [16-glutamic acid, 25-histidine, 27-glycine, 28-glycine, 29-glycine, 30-glycine] (human B-chain) fusion protein with peptide (synthetic 7-amino acid linker) fusion protein with insulin [47-threonine, 51-aspartic acid, 58-glycine] (human A-chain) fusion protein with peptide (synthetic 20-amino acid linker) fusion protein with immunoglobulin G2 (human Fc fragment), dimer; and (2) *Homo sapiens* Insulin B-chain [Y16>Y(16), F25>H(25), TPKT27-30>GGGG(27-30)] (1-30) fusion protein with diglycylseryltetraglycyl (31-37) Insulin A-chain [I10>T(47), Y14>D(51), N21>G(58)] (38-58) fusion protein with tris(tetraglycylglutaminyl)pentaglycyl (59-78) *Homo sapiens* Immunoglobulin heavy constant gamma 2 {del-CH1, hinge-(7-12), CH2, CH3[K¹⁰⁷>del(300)]} (79-299), dimer (80-80':83-83')-bisdisulfide, expressed in CHO cells, alfa glycosylated.

Each monomer of BIF has the amino acid sequence set forth in SEQ ID NO:1: 
FVNQHLCGSHLVEALELVCGERGFHYGGGGGGSGGGGIVEQCCTSTCSL
DQLENYCGGGGGQGGGGQGGGGQGGGGGECPPCPAPPVAGPSVFLFPPKP
KDTLMISRTPEVTCVVVDVSHEDPEVQFNWYVDGVEVHNAKTKPREEQFN
STFRVVSVLTVVHQDWLNGKEYKCKVSNKGLPAPIEKTISKTKGQPREPQ
VYTLPPSREEMTKNQVSLTCLVKGFYPSDIAVEWESNGQPENNYKTTPPM
LDSDGSFFLYSKLTVDKSRWQQGNVFSCSVMHEALHNHYTQKSLSLSPG
 (SEQ ID NO:1). Each monomer includes intrachain disulfide bonds between cysteine residues at positions 7 and 44, 19 and 57, 43 and 48, 114 and 174 and 220 and 278. The two monomers are attached by disulfide bonds between the cysteine residues at positions 80 and 83 to form the dimer. BIF's structure, function and production are described in more detail in U.S. Patent Application Publication No. 2016/0324932.

When used herein, the term "BIF" refers to any insulin receptor agonist comprised of two monomers having the amino acid sequence of SEQ ID NO:1, including any protein that is the subject of a regulatory submission seeking approval of an insulin receptor agonist product that relies in whole or part upon data submitted to a regulatory agency by Eli Lilly and Company relating to BIF, regardless of whether the party seeking approval of said product actually identifies the insulin receptor agonist as BIF or uses some other term.

Embodiments of criteria and guidelines for determining weekly maintenance doses are described in more detail below.

Administration of Loading Dose.With a long elimination half-life, administration of once-weekly insulin receptor agonists suitable for once weekly dosing, following a weekly dosing regimen could result in pharmacokinetic steady-state not being reached for multiple weeks. In addition, the long elimination half-life of such products when administered once-weekly for multiple weeks may result in peak concentration levels significantly greater than the peak serum concentration level that would be observed after administration of a single dose. Uses described herein address these issues through administration of a single initial one-time-only loading dose designed to reduce the time to reach pharmacokinetic steady state. Thus, when used herein, the term "loading dose" refers to a first dose of long-acting insulin receptor agonist suitable for once-weekly dosing administered to a given subject that is larger than the dose that would be expected to be used for long-term of maintenance treatment.

The loading doses described herein generally comprise a single dose that is greater than the expected weekly maintenance dose, described in more detail below, by a factor that allows a near steady-state concentration to be reached within the first week after administering the single dose.

For BIF, steady-state serum concentration levels following initiation of once-weekly dosing is predicted to be reached after approximately 8 to 10 or 12 weeks of once-weekly administration without a loading dose. A near steady-state concentration can be reached within the first week of dosing when using a loading dose approximately 3-fold higher than the initial weekly maintenance dose or expected weekly maintenance dose.

Because such a subject's daily basal insulin doses are being replaced by a single dose each week, the daily dose in units must be translated into a weekly dose, referred to herein as the "daily basal dose weekly equivalent," that would be expected to provide the same level of insulin activity each day upon reaching steady-state through once-weekly dosing. The daily basal dose weekly equivalent could be expressed in insulin units or mg. If expressed in insulin units, the daily dose of units of once-daily basal insulin prior to initiation of treatment with a once-weekly insulin receptor agonist would be multiplied by 7 in order to obtain the daily basal dose weekly equivalent of once-weekly insulin receptor agonist. For example the daily basal dose weekly equivalent for a patient who had been receiving 42 units / day of insulin degludec prior to initiation of treatment with a once-weekly insulin receptor agonist would by 294 units.

In certain embodiments, the once-weekly insulin receptor agonist is provided in a device that allows for adjustment in 5- or 10-unit increments, so when determining doses of once-weekly insulin to be administered, the daily basal dose weekly equivalent calculated as described above would be rounded to the nearest 5 or 10 units. Thus, in certain embodiments when the once weekly insulin is provided in a device allowing for dose adjustments in 5-unit increments, a daily basal dose weekly equivalent of 294 units, calculated as described above, could be rounded to 295 units or 290 units.

If expressed in mg, the dose in units must be converted to the mg equivalent based on the potency of the insulin receptor agonist suitable for once weekly dosing using what is referred to herein as either a "weekly basal conversion factor," or a "daily basal conversion factor." For example, one mg of BIF has been determined to provide about 35 units over the course of a week, so the weekly basal conversion factor of BIF has been determined to be about 35 U/week/mg and the daily basal conversion factor of BIF has been determined to be about 5 U/day/mg. Thus, the mg dose of BIF for a patient who had been receiving 42 units per day of insulin degludec would be about 8.4 mg (42 units/day x 7 days = 294 units/week / 35 U/week/mg).

In certain embodiments, the determination of a daily basal dose weekly equivalent may require additional adjustments for patients being treated with certain basal insulins. For example, for patients being treated with neutral protamine Hagedorn (NPH) insulin twice-daily, in certain embodiments their daily basal insulin dose should be reduced by 20% before determining their daily basal dose weekly equivalent as described above. Similarly, for patients being treated with U300 insulin glargine, in certain embodiments their daily basal insulin dose should be reduced by 20% before determining their daily basal dose weekly equivalent as described above.

In certain embodiments, the uses described herein are designed to minimize the time until a subject reaches acceptable glycemic control by providing a loading dose.

### Determination and Administration of Weekly Maintenance Doses.

When used herein, the term "weekly maintenance dose" refers to any single weekly dose of insulin receptor agonist suitable for once-weekly dosing other than a loading dose, as defined above.

In certain embodiments, weekly maintenance doses may be specifically identified herein according to the number of previous doses of insulin receptor agonist that have been administered. For example, use herein of the term "first weekly maintenance dose" refers to either the weekly maintenance dose administered one week after the loading dose was administered. Use herein of the term "second weekly maintenance dose" refers to the weekly maintenance dose administered the week after the "first weekly maintenance dose." Use herein of the term "third weekly maintenance dose" refers to the weekly maintenance dose administered the week after the "second weekly maintenance dose."

In certain embodiments, the uses described herein provide for determination and administration of one or more weekly maintenance doses, and are designed to enable patients to reach target FG levels after administration of as few doses as possible while minimizing the risk of hypoglycemia.

The dose regimens herein vary in their complexity, with less complex regimens providing advantages on ease of interpretation and implementation, and more complex adjustments and regimens providing potential advantages in terms of glucose control. In general, however, the regimens described herein share certain common features: dose decreases when FG is low; no dose adjustments when FG is at or near target; and dose increases when FG is high. In addition, in certain embodiments the magnitude of dose adjustment generally depends on how far the patient's FG is from target - *e.g.,* FG well above or below target would result in a larger adjustment than FG slightly above or below target.

In certain embodiments wherein the patient has T2D and has not yet progressed to the need for MDI treatment, the initial weekly maintenance dose is either 100 U, for insulin naive patients, or is equal to the daily basal dose weekly equivalent described above for patients currently being treated with a once-daily basal insulin (i.e., 7x the patient's current daily dose of basal insulin). For subsequent weekly maintenance doses, determining whether dose adjustments are needed will be determined based on the patient's FG levels.

In certain embodiments, adjustments to weekly maintenance doses may be determined according to the guidelines set forth below in Table 6:

**Table 6.**

| Median FG Value (mg/dL) | Dose Adjustment (Units) |
|---|---|
| <80 | -20 |
| 80-120 | No change |
| 121-140 | +20 |
| >140 | +40 |

In other embodiments, in particular for T2DM patients who are switching from a once-daily basal insulin but have not progressed to MDI treatment and either have median FG ≤120 mg/dL prior to initiation of treatment with a once-weekly insulin and/or a once daily basal insulin dose of <20 units/day, adjustments to weekly maintenance doses may be determined according to the guidelines set forth below in Table 7.

**Table 7.**

| Median FG Value (mg/dL) | Dose Adjustment (Units) |
|---|---|
| <80 | -20 |
| 80-120 | No change |
| 121-140 | +10 |
| >140 | +20 |

In other embodiments, in particular for patients who are switching from a once-daily basal insulin and have a once daily basal insulin dose of <10 units/day, adjustments to weekly maintenance doses may be determined according to the guidelines set forth below in Table 8.

**Table 8.**

| Median FBG | | BIF Dose adjustment |
|---|---|---|
| mg/dL | Mmol/L | Units |
| <80 | <4.4 | (-) 20 units (decrease) |
| 80-120 | 4.4-6.6 | No change to dose |
| 121-140 | 6.7-7.7 | (+) 5 units (increase) |
| >140 | >7.7 | (+) 10 units (increase) |

In addition, any adjustments to weekly maintenance doses described above typically take into account any incidents of hypoglycemia. For example, if a patient experienced any episode of hypoglycemia as indicated by a blood glucose of <70 mg/dL in the previous week, his or her dose should not be increased, even with a median FG falling into one of the ranges for which a dose adjustment would be recommended. Further, certain criteria may lead to a recommended decrease in the dose. For example, in certain embodiments, a dose reduction of 40 units is recommended for a patient who meets any of the criteria set forth in Table 9 below.

**Table 9. Exemplary hypoglycemia-based dose decreases.**

| Hypoglycemia Dose Reduction Criteria Based on BG Values | Unit Dose Decrease |
|---|---|
| ≥ 3 episodes ≤ 70 mg/dL | 40 units |
| ≥ 1 nocturnal episode ≤ 70 mg/dL | |
| ≥ 1 episode ≤ 54 mg/dL | |
| any confirmed severe hypoglycemia | |

In certain embodiments for T2D patients being treated with MDI and who are switching from once-daily basal insulin to a once-weekly insulin, adjustments to weekly maintenance doses may be determined according to the guidelines set forth below in Table 10.

**Table 10.**

| Median FBG (mg/dL) | BIF adjustment (units) | |
|---|---|---|
| | Baseline | Baseline |
| | FG ≤ 120 mg/dL and/or daily basal dose < 20 units | FG > 120 and daily basal dose ≥ 20 units |
| <80 | -10 to 20 | -20 |
| 80-120 | No change | No change |
| 121-140 | +10 | +20 |
| > 140 | +20 | +40 |

For T1D patients, similar to the loading dose calculations described above in Table 10, in certain embodiments, determination of the patient's first weekly maintenance dose depends on the patient's baseline FG prior to initiation of treatment with the once weekly insulin.

For example, in certain embodiments, the first weekly maintenance dose for a T1D patient having FG ≤ 140 mg/dL, the loading dose would be 7x greater than their daily basal dose, but for patients having baseline FG 141-160 or > 160, their daily basal dose would be increased by about 10-20% or about 20-30%, respectively, before converting to a weekly dose, as depicted in Table 11 below.

**Table 11. Dose adjustments for first weekly maintenance dose for T1D patients.**

| Baseline FBG | First weekly maintenance dose |
|---|---|
| ≤ 140 mg/dL | Prior daily dose (U) x 7 |
| 141 - 160 mg/dL | [Prior daily dose x 1.1 to 1.2; increase dose by 10 to 20%] x 7 |
| > 160 mg/dL | [Prior daily dose x 1.2 to 1.3; increase dose by 20 to 30%] x 7 |

Adjustments for subsequent weekly maintenance doses for T1D patients in certain embodiments are determined according to the patient's FG and current dose of once-weekly insulin. For example, the upwards adjustment in dose of patients having FG above the target glucose range may be more aggressive for patients that received a dose above a certain threshold, and less aggressive for patients that received a dose below a certain threshold. In certain embodiments, adjustments to weekly maintenance doses for T1D patients may be determined according to the guidelines set forth below in Table 12.

**Table 12. Dose adjustments for subsequent weekly maintenance doses for T1D patients.**

| Median FPG (mg/dL) | Adjustment | |
|---|---|---|
| | Current dose < 100 U | Current dose > 100 U |
| < 80 | Previous lower dose | Previous lower dose |
| 80-120 | No change | No change |
| 121-150 | + 5 U | +10U |
| 151-180 | + 10 U | +20U |
| >180 | +20U | +30U |

The dose adjustments described above in Tables 11 and 12 may also be subject to caveats for patients who experienced hypoglycemia events. Although the hypoglycemia-based limitations on dose adjustments described above for T2D patients not being treated with MDI may in some cases also be appropriate for T1D and T2D patients being treated with MDI, those limitations may result in some cases in underdosing of weekly basal insulin, because such patients may experience hypoglycemia associated with their MDI treatment and/or disease characteristics that would unnecessarily trigger dose reductions or limitations on dose increases of their basal insulin. Thus, in certain embodiments, for patients being treated with MDI, their once-weekly basal dose should be reduced to the previous lower dose if the patient experienced hypoglycemia believed to be attributed to the once-weekly basal insulin, rather than the patient's prandial insulin. For example, a reduction to the previous lower dose may be indicated in some embodiments if the patient had any incident of nocturnal hypoglycemia. For example, in such embodiments, even if a patient's FBG from the preceding week was greater than 120 and a dose increase would be indicated according to the guidelines set forth above in Tables 11 or 12, if the patient had hypoglycemia warranting a reduction in dose of basal insulin, their dose of once weekly insulin would be decreased to the previous lower dose they had received prior to the dose administered the preceding week. In certain embodiments, if the patient had no previous dose because this was the first assigned dose, then the daily basal dose weekly equivalent may be decreased, for example by about 10-20%. Similarly, if the patient had been receiving the same weekly maintenance dose since start of treatment, the previous week's dose may be decreased by about 10-20%.

In certain embodiments that may be applicable to a broad spectrum of patients, adjustments to weekly maintenance doses may be determined according to the guidelines set forth below in Table 13:

**Table 13.**

| FG Value (mg/dL) | Dose Adjustment (Units) |
|---|---|
| <54 | -28 |
| 55-70 | -14 |
| 71-100 | No Change |
| 101-125 | 14 |
| >125 | 28 |

In addition, in certain embodiments such as those set forth in the guidelines above in Table 5, dose decreases are implemented if any of the following occur: multiple episodes of recorded hypoglycemia with SMBG <70 mg/dL; severe hypoglycemia (requiring assistance); or documented hypoglycemia ≤54 mg/dL in the preceding week. In certain embodiments, the dose may not be increased if any individual blood glucose reading was documented at <70 mg/dL at any time in the preceding week.

The guidelines described above may also be expressed in the form of the following equation: $Weekly maintenance dose = previous dose - 14 * X + 14 * Y$ wherein:
X = 0 if FG is >71;
X = 1 if FG is 55-70; or
X = 2 if FG is <54; and
wherein:
   Y = 0 if FG ≤100;
   Y = 1 if FG is 101-125; or
   Y = 2 if FG is >125;
   with the proviso that Y may not be > 0 if the subject had any SMBG reading <70 mg/dL at any time in the preceding week.

In other embodiments, the FG target is 120 mg/dL and the criteria for dose adjustments are set forth in Table 14 below:

**Table 14.**

| Criteria used to determine dose | Dose adjustment criteria |
|---|---|
| < 70mg/dL | - 2 mg |
| 71 to 99 mg/dL | - 1.4 mg |
| 100 to 119 mg/dL | 0 mg |
| 120 to 139 mg/dL | 0 mg |
| 140-180 mg/dL | + 1.4 mg |
| > 180 mg/dL | + 2 mg |
| Number of hypoglycemic episodes to trigger dose reduction | 1 |
| Dose reduced by | - 1.4 mg |
| Dose adjustment interval | 14 days |

In other embodiments, the FG target is 140 mg/dL, and the criteria for dose adjustments are set forth in Table 15 below:

**Table 15.**

| Criteria used to determine dose | Dose adjustment criteria for target FG 140 mg/dL |
|---|---|
| < 70mg/dL | - 2 mg |
| 71 to 99 mg/dL | - 1.4 mg |
| 100 to 119 mg/dL | 0 mg |
| 120 to 139 mg/dL | + 1 mg |
| 140-180 mg/dL | + 1.4 mg |
| > 180 mg/dL | + 3 mg |
| Number of hypoglycemic episodes to trigger dose reduction | 1 |
| Dose reduced by | - 1.4 mg |
| Dose adjustment interval | 30 days |

**In** certain embodiments, determination of weekly maintenance doses may also take into consideration the number of previous doses of insulin receptor agonist the patient has been administered. While the complexity of such regimens may vary, in general the magnitude of recommended dose adjustment is relatively greater for the first weekly maintenance dose administered to a patient having certain characteristics than for the second or subsequent weekly maintenance doses.

**In** certain embodiments, adjustments to weekly maintenance doses may be determined according to the guidelines set forth below in Table 16:

**Table 16.**

| **Median FG value from 3 days (mg/DL)** | **Dose adjustment based on previous weeks insulin dose (Units)** | | |
|---|---|---|---|
| | **Week 2** | **Week 3** | **Week 4 and subsequent** |
| ≤80 | -70 | -70 | -14 |
| 81-100 | -42 | -28 | No change |
| 101-140 | No change | No change | 14 |
| 141-180 | 120 | 70 | 28 |
| >180 | 210 | 120 | 42 |

In addition to the guidelines in Table 16, dose decreases will be implemented based on the occurrence of any of the following instances of hypoglycemia: multiple episodes of recorded hypoglycemia with SMBG <70 mg/dL; severe hypoglycemia (requiring assistance); and/or documented hypoglycemia ≤54 mg/dL in the preceding week. In addition, the dose may not be increased if any SMBG reading was documented at <70 mg/dL at any time in the preceding week.

In certain embodiments, the guidelines for adjustment of weekly maintenance doses set forth in Table 16 above are implemented in the form of the following equation:$Weekly maintenance dose = previous dose - 14 ∗ X + 14 ∗ Y$ wherein:
X = 0 if either: the subject's FG is ≥ 101; or the subject has been administered at least 3 previous doses and has FG 81-100;
X = 1 if the subject's FG is ≤80 and the subject has been administered at least 3 previous doses;
X = 2 if the subject's FG is 81-100 and the subject has been administered two previous doses;
X = 3 if the subject's BW is 81-100 and the subject has been administered one previous dose; or
X= 5 if the subject's median FG is ≤80 and the subject has been administered one previous dose; and
wherein:
Y = 0 if either: the subject's FG is ≤ 100; or the subject's FG is 81-100 and the subject has been administered at least 3 previous doses;
Y = 1 if the subject's FG is 101-140 and the subject has been administered at least 3 previous doses;
Y = 2 if the subject's FG is 141-180 and the subject has been administered at least 3 previous doses;
Y = 3 if the subject's FG is >180 mg/dL and the subject has been administered at least 3 previous doses;
Y = 5 if the subject's FG is 141-180 and the subject has been administered 2 previous doses;
Y = 8.57 if either: the subject's FG is 141-180 and the subject has been administered 1 previous dose; or the subject's FG is >180 and the subject has been administered 2 previous doses; or
Y = 15 if the subject's FG is >180 and the subject has been administered 1 previous dose;
with the proviso that Y may not be > 0 if the subject had any SMBG reading <70 mg/dL at any time in the preceding week.

In certain embodiments, the adjustments recommended for weekly maintenance doses to be administered each week may be described through reference to addition or subtraction of a given fraction or quantity of "dose adjustment units," which represents the smallest dose adjustment recommended for that particular insulin. For example, if the smallest dose adjustment recommended for any patient being treated with a given insulin receptor agonist is 2 IU, an increase of 4 dose adjustment units would refer to an increase of 8 IU. In certain embodiments, the dose adjustment unit is between about 0.5-5 IU. In other embodiments the dose adjustment unit is between about 0.75-4 IU. In other embodiments the dose adjustment unit is between about 1-3 IU. In a preferred embodiment the dose adjustment unit is 1.75 IU. Dose adjustment units may also be expressed in other units of measure, such as mg.

In certain embodiments, the regimens, uses and methods described herein are designed to achieve FG of 100 mg/dL and comprise 5 ranges based on these patient characteristics: (1) < about 80 mg/dL; (2) between about 80 to - about 100 mg/dL; (3) between about 101 - about 140 mg/dL; (4) between about 141 - about 180 mg/Dl; and (5) > about 180 mg/dL. For patients in the first range who have switched from a daily basal insulin, the recommended dose adjustment is a decrease for each of the first, second and third (or any subsequent) weekly maintenance doses, but the amount by which the dose is decreased is reduced for the second and third (or any subsequent) weekly maintenance doses by 30% and 50%, respectively, as compared to the amount by which the dose is decreased for the first weekly maintenance dose. For patients in the second range who have switched from a daily basal insulin, the recommended dose adjustment is a decrease for the first and second weekly maintenance doses, wherein the amount by which the dose is decreased is reduced for the second weekly maintenance dose by 50% as compared to the amount by which the dose is decreased for the first weekly maintenance dose, and no dose adjustment is recommended for the third (or any subsequent weekly maintenance doses). For patients in the third range who have switched from a daily basal insulin, no adjustment is recommended for the first and second weekly maintenance doses, and an upward adjustment is recommended for third (or any subsequent) weekly maintenance dose. For patients in the fourth range who have switched from a daily basal insulin, the recommended dose adjustments are increases for the first, second and third (or any subsequent) weekly maintenance dose, but wherein the amount of increase for the second and third (or any subsequent) weekly maintenance dose decreases by 50% as compared to the amount of increase for the first weekly maintenance dose. Finally, for patients in the fifth range, the recommended dose adjustments are increases for the first, second and third (or any subsequent) weekly maintenance dose, but wherein the amount of increase for the second and third (or any subsequent) weekly maintenance dose decreases by 50% and 75%, respectively, as compared to the amount of increase for the first weekly maintenance dose.

For example, for patients who have switched from a daily basal insulin to BIF, the recommended dose adjustments in certain embodiments are set forth in Table 17 below:

**Table 17. D = previous dose.**

| **Median Fasting glucose (mg/dL)^{a}** | **Median Fasting glucose (mmol/L)** | **Week 1 (Visit 4) (2^{nd} dose)** | **Week 2 (Visit 5) (3^{rd} dose)** | **Week 3 (Visit 6) and all Subsequent Weeks** |
|---|---|---|---|---|
| <80 mg/dL or have any nocturnal hypoglycemia or multiple (≥3) episodes of hypoglycemia | <4.4 mmol/L or have any nocturnal hypoglycemia or multiple (≥3) episodes of hypoglycemia | D - 2 mg | D - 1.4 mg | D - 1 mg |
| 80 - 100 mg/dL | 4.4 - 5.5 | D - 1.5 mg | D - 1 mg | D |
| 101 - 140 mg/dL | 5.6 - 7.7 | D | D | D + 0.5 mg |
| 141 - 180 mg/dL | 7.8 - 10.0 | D+ 1.5 mg | D + 1 mg | D + 0.5 mg |
| >180 mg/dL | >10.0 | D + 3 mg | D + 1.5 mg | D + 1 mg |

In certain embodiments, the dose adjustments for weekly maintenance doses for insulin-naive patients targeting FG of 100 mg/dL follow the same general principles as described above, but with some differences in specific application of those principles, such as different magnitudes of adjustments and distinctions in recommended adjustments, in some cases, depending on the size of the previous dose. In certain embodiments, the recommended dose adjustments for insulin-naive patients who initiate basal insulin treatment on BIF are set forth in Table 18 below:

**Table 18. D = previous dose.**

| **Median FG (mg/dL)** | **Median FG (mmol/L)** | **Week 1 (Visit 4) (2^{nd} dose)** | **Week 2 (Visit 5) (3^{rd} dose)** | **Subsequent Week** | |
|---|---|---|---|---|---|
| | | | | **Dose ≤5 mg** | **Dose >5 mg** |
| < 80 mg/dL or have any nocturnal hypoglycemia or multiple episodes of hypoglycemia | < 4.4 mmol/L or have any nocturnal hypoglycemia or multiple episodes of hypoglycemia | D - 2 mg | D - 2 mg | D - 2 mg | D-3 mg |
| 80 - 100 mg/dL | 4.4 - 5.5 | D - 1.5 mg | D - 1 mg | No change | No change |
| 101 - 140 mg/dL | 5.6 - 7.7 | No change | No change | D + 0.5 mg | D + 1 mg |
| 141 - 180 mg/dL | 7.8 - 10.0 | D + 4 mg | D + 2 mg | D + 1 mg | D + 1.5 mg |
| > 180 mg/dL | > 10.0 | D + 7 mg | D + 4 mg | D + 1.5 mg | D+2mg |

In certain embodiments, after dose adjustments determined according to the criteria described above have been administered for a certain number of weeks, patients will reach a level of serum glucose control for which the need for dose adjustment may be determined less frequently than once a week. For example, in certain embodiments, a patient will use the criteria described above to determine dose adjustments once a week for the first 8, 9, 10, 11 or 12 weeks of treatment, but thereafter will only determine whether an adjustment is needed every 2, 3 or 4 weeks. In certain preferred embodiments, a patient will use the criteria described above to determine dose adjustments once a week for the first 12 weeks of treatment, but thereafter will only determine whether an adjustment is needed every 4 weeks. Even in these embodiments, however, incidents of hypoglycemia may preclude dose increases or lead to dose decreases according to the criteria described above.

Although certain of the embodiments described above are described for use with particular patient populations defined by certain criteria, such as whether the patient has T1D or T2D, the patient's baseline FG, whether the patient is being treated with a daily basal insulin, the patient's current dose of basal insulin, and whether the patient is being treated with MDI, use of such embodiments need not be mutually exclusive to that population. Thus, in some cases aspects of guidelines described above for one population may be used as appropriate for determination of doses and dose adjustments for other populations as well.

In certain embodiments, the doses described herein are administered from a reusable pen injector, or a disposable pen device.

### Other definitions.

When used herein, the terms "approximately" and "about" are intended to refer to an acceptable degree of error for the amount or quantity indicated given the nature or precision of the measurements. For example, the degree of error can be indicated by the number of significant figures provided for the measurement, as is understood in the art, and includes but is not limited to a variation of +/-1 in the most precise significant figure reported for the amount or quantity. Typical exemplary degrees of error are within 20 percent (%), preferably within 10%, and more preferably within 5% of a given value or range of values. Numerical quantities given herein are approximate unless stated otherwise, meaning that the term "about" can be inferred when not expressly stated.

When used herein, the term "dose" or "doses" refers to the quantity of insulin receptor agonist suitable for once weekly dosing that is administered to an individual in discrete amount at a particular point in time. When used in connection with the terms dose, dosing, doses and the like, the term "adjustment" refers to the quantity of any decrease or increase to the dose administered the prior week. When used in connection with the terms dose, dosing, doses and the like, the term "regimen" refers to a set of guidelines for determining and administering one or more doses and/or adjustments thereto.

When used herein, the term "baseline" refers to characteristics of a patient prior to initiation of treatment with an insulin receptor agonist suitable for once weekly dosing. For example, a patient's baseline FG is his or her FG before the initial dose of insulin receptor agonist suitable for once weekly dosing is administered.

When used herein, the terms "fasting glucose," "FG," "fasting blood glucose," "FBG," "fasting plasma glucose" or "FPG" refer to plasma glucose level from a sample of blood taken or obtained via continuous glucose monitoring (CGM) after a patient fasts overnight. When used in the context of determining the dose of insulin receptor agonist suitable for once weekly dosing to be administered to a patient, unless otherwise specified herein, the patient's FG is determined as the median FG from multiple days, typically at least 3 days and no more than 7 days.

When used herein, the terms "treatment," "treat," "treating," and the like, are meant to include slowing or attenuating the progression of a disease or disorder. These terms also include alleviating, ameliorating, attenuating, eliminating, or reducing one or more symptoms of a disorder or condition, even if the disorder or condition is not actually eliminated and even if progression of the disorder or condition is not itself slowed or reversed.

Treatment with "MDI" refers to treatment with injections of basal insulin in combination with a bolus or prandial insulin. Bolus or prandial insulins are shorter-acting and typically given at mealtime. Examples of bolus or prandial insulins used in such regimens include insulin lispro, insulin aspart, insulin glulisine, and regular insulin.

A "subject" refers to a mammal, preferably a human with a disease, disorder or condition that would benefit from treatment with an insulin receptor agonist suitable for once weekly dosing.

"Glycemic control" refers to a subject's blood sugar levels, as measured for example by blood glucose and/or HbA1c levels; "providing" glycemic control refers to maintaining or improving glycemic control; "maintaining" glycemic control refers to maintaining the time with blood glucose levels within a target range and/or maintaining or reducing HbA1c; "improving" glycemic control refers to increasing the time with blood glucose levels within a target range and/or reductions in HbA1c; and "in need of further" glycemic control refers to a need for an increased time with blood glucose levels in a target range and/or reductions in HbA1c.

"HbA1c" refers to glycated hemoglobin levels, which develop when hemoglobin joins with glucose in the blood. HbA1c levels are a commonly used measure of glycemic control in patients with diabetes.

"Hypoglycemia" refers to low blood sugar, and an "episode" of hypoglycemia refers to an instance of low blood sugar, as observed for example in a plasma glucose test or value from a personal blood glucose meter (BGM) or CGM device, in many cases less than about 70 mg/dL.

An episode of "severe" hypoglycemia is a severe event characterized by altered mental and/or physical status requiring assistance for treatment of hypoglycemia. For example, a subject with altered mental status, and could not assist in their own care, or was semiconscious or unconscious, or experienced coma with or without seizures, and the assistance of another person was needed to actively administer carbohydrate, glucagon, or other resuscitative actions. Glucose measurements may not be available during such an event, but neurological recovery attributable to the restoration of glucose concentration to normal is considered sufficient evidence that the event was induced by a low glucose concentration.

The uses described herein may be provided in simultaneous or sequential combination with other T2D treatments, including oral T2D medications such as metformin, and/or other injectable medications including rapid-acting or basal insulins or GLP-1 receptor agonists.

The invention is further illustrated by the following examples.

### EXAMPLES

### SAD and MAD Studies.

Studies are designed to evaluate the pharmacokinetics (PK) and pharmacodynamics (PD) effect of BIF in healthy volunteers and patients with T2DM. The single ascending dose (SAD) study is randomized, investigator- and subject-blind, placebo-controlled, single-dose, single site, dose-escalation study conducted in healthy subjects and patients with T2D. Six dose levels of BIF -- 2, 10, 12, 17, 20, and 35 mgare explored in the study. The dose escalation of BIF is assessed in cohorts of 8 healthy subjects or 8 patients with T2D (6 patients on BIF and 2 patients on placebo per cohort). Staggered dosing is performed for each cohort, in which a new higher dose of BIF was administered. Since the unit dose of BIF required to achieve glycemic control equivalent to 1 unit of basal insulin was not known, the dose of BIF is expressed in milligrams. Blood samples are collected pre-dose, and 8 hours post-dose on Day 1 and once a day on Days 2-7, Day 14 and at the follow-up visit (Day 29).

Plasma samples obtained during this study are analyzed for BIF using a validated enzyme-linked immunosorbent assay (ELISA) method. The lower limit of quantification (LLOQ) is 300.00 pM, and the upper limit of quantification is 10000.00 pM. The inter-assay accuracy (percent [%] relative error) during validation ranged from -1.0% to 5.4%. The inter-assay precision (% coefficient of variation [CV]) during validation ranges from 4.8% to 9.6%.

Plasma samples are analyzed for insulin glargine, using a validated high-performance liquid chromatography method and tandem mass spectrometry (MS/MS) detection. The LLOQ for insulin glargine is 8.25 pM. The upper limit of quantification for insulin glargine is 1649.4 pM. The inter-assay accuracy during validation for insulin glargine, respectively. The inter-assay precision (% relative standard deviation) during validation for insulin glargine ranges from 3.8% to 9.0%.

The full analysis set used for PK and PK/PD analysis consists of all enrolled patients/subjects receiving at least 1 dose of the study drug, according to the treatment the patients/subjects received. PK parameter estimates for BIF and insulin glargine and metabolites are calculated by standard noncompartmental methods of analysis using Phoenix WinNonlin version 6.4 (Pharsight Corporation, USA). Analysis of PD data used S-Plus for Windows version 8.2.

The primary parameters for analysis are time to maximum BIF concentration (tₘₐₓ), BIF half-life, and BIF peak-to-trough ratio. PD analysis for patients with T2D are performed, based on FG (using the pre-breakfast blood fasting glucose from the 8-point glucose profiles) version 7.3.

57 patients with T2D and 16 healthy subjects participate in the study. All 16 healthy subjects completed the study. Of the 56 patients with T2D that complete the study, 36 receive BIF (6 each received 2, 10, 12, 17, 20, and 35 mg), 12 receive placebo, and 8 receive insulin glargine. Of the 16 healthy subjects, 12 receive BIF (6 each received 5 and 10 mg) and 4 receive placebo.

The PK results from the SAD study include all 36 patients with T2D and 10 healthy subjects that receive BIF. The dose-response is linear in healthy subjects and patients, and there is low between-day and between-subject variability in patients with T2D. On average, after a single-dose administration, BIF reaches Cₘₐₓ on Day 4. The median (range) tₘₐₓ for patients receiving 2 mg, 10 mg, 12 mg, 17 mg, 20 mg, and 35 mg is 4.5 (3.0-6.0), 4.5 (3.0-5.0), 4.0 (3.0-5.0), 4.5 (2.0-14.0), 4.0 (4.0-5.0), and 4.5 (3.0-6.0), respectively. The median (range) tₘₐₓ for healthy subjects receiving 5 mg or 10 mg is 3.0 (3.0-4.0) and 3.0 (1.0-6.0), respectively. The maximum concentration appears to increase proportionally with dose in healthy subjects and patients with T2D. The BIF plasma concentration declines with a mean half-life of approximately 17 days in patients with T2D. The calculated half-life ranges from 14.8 to 18.5 days for the different BIF doses in patients with T2D and 11.8 to 15.5 days for the 2 BIF doses in healthy subjects.

The PD results from the SAD study indicate that a single-dose administration of BIF in patients with T2D led to a decrease in fasting glucose that was sustained until at least 5 days post-dose. BIF administration resulted in glucose-lowering within the first day of administration. There is greater reduction in FG with increasing BIF concentrations.

Data from the SAD study demonstrates clear evidence of glucose lowering in study participants with T2DM following single doses of BIF ranging from 2 to 35 mg. The PK of BIF following single doses demonstrated prolonged time-action profile to support once-weekly administration. BIF reached maximum concentration approximately 4 days after dosing, followed by a mean elimination half-life of approximately 17 days (ranging from 11 to 22 days) in study participants with T2DM.

With a long elimination half-life, following a weekly fixed-dose regimen, PK steady-state was predicted to be reached in approximately 8 to 10 weeks with approximately 3-fold higher concentration due to accumulation than after single dose. Therefore, based on PK modeling a loading dose strategy 3 times the weekly dose would achieve steady-state exposure after 1 dose.

The 3X loading dose strategy was employed in a multiple-ascending dose (MAD) study. The MAD study is a three-site, randomized, open-label, active-controlled, multiple-dose, 2-part, dose-escalation and parallel design study in patients with T2D conducted to assess the safety and tolerability of BIF. Dose escalation of BIF is assessed in cohorts of up to 8 patients with T2D (6 BIF and 2 insulin glargine [Sanofi; Paris, France]). Patients on insulin glargine (U100) continue their usual dosing regimen until pre-dose on Day 1 and patients previously not treated with insulin glargine are converted to an appropriate insulin glargine dose at the judgement of the investigator. Patients assigned to the BIF group receive a loading dose of 3 times the weekly maintenance dose during the first week administration at day 1, and a weekly maintenance dose (1, 2, 5, and 10 mg) once weekly for the following 5 weeks to achieve quickly steady state concentration. Patients are discharged from the CRU on Day 8. Patients assigned to the insulin glargine control group administer daily insulin glargine injections at the same dose and timing as their usual basal insulin.

Plasma samples obtained during the study are analyzed for BIF and insulin glargine, and PK and PK/PD analyses were conducted as described above for the SAD study.

33 patients with T2D participate in the study, 28 completing the study. The patients are between 40 and 69 years old and 18 were males (Table 1). Twenty-five patients receive BIF on Day 1 (7 received 1 mg, 6 received 2 mg, 6 received 5 mg, and 6 received 10 mg) and 22 receive BIF on Week 6 (6 received 1 mg, 5 received 2 mg, 5 received 5 mg, and 6 received 10 mg).

Data indicate that, after a loading dose in Week 1, the overall mean BIF tₘₐₓ was 4.3 days across all doses studied. PK following a single loading dose is comparable to concentration profile at Week 6 (at 1/3 dose delivered once weekly), indicating successful transition of patients to PK profile similar to steady state and supporting the loading dose strategy. In the patients with T2D, the peak-to-trough ratio of dose-normalized BIF concentrations over a 1-week period is ~1.14 in steady state.

Mean 7-point glucose profiles are measured on Day -1, 4, and 40. The glucose profiles remain constant over time across the 4 BIF treatment groups. Specifically, the glucose profiles of patients receiving BIF weekly are consistent over the 6-week study. Furthermore, the BIF 7-point glucose profiles are similar to the insulin glargine profiles. Higher dose levels elicit greater glucose responses. There are no significant differences between subjects receiving BIF and insulin glargine.

Hypoglycemia is the most frequent treatment-related adverse reaction. There were fewer episodes reported following BIF administration compared to insulin glargine (44.0% versus 62.5%). Most of the events were treated with food or drink.

### Phase 2 Studies.

### T2DM Patients Previously Treated with Oral Antidiabetic Drugs and a Basal Insulin

A study is designed to evaluate the safety and efficacy of BIF compared to insulin degludec over 32 weeks in patients with T2DM previously treated with oral antidiabetic drugs and a basal insulin. The study design includes a loading dose and 2 different algorithms for determination of BIF weekly maintenance doses.

The loading dose and expected weekly maintenance dose are determined according to Table 19 below:

**Table 19. Abbreviations: IU = international units; HbA1c = hemoglobin A1c**

| **Daily basal insulin dose prior to randomization** | **Baseline HbA1c (%)** | **Loading dose (mg)** | **Expected Weekly maintenance dose (mg)** |
|---|---|---|---|
| 10 IU - 19 IU | <8.5 | 6 | 4 |
| 20 IU - 29 IU | <8.5 | 10 | 6 |
| 30 IU - 39 IU | <8.5 | 12 | 8 |
| 40 IU - 50 IU | <8.5 | 18 | 10 |
| >50 IU | <8.5 | 24 | 12 |
| 10 IU - 19 IU | ≥8.5 | 12 | 4 |
| 20 IU - 29 IU | ≥8.5 | 18 | 6 |
| 30 IU - 39 IU | ≥8.5 | 24 | 8 |
| 40 IU - 50 IU | ≥8.5 | 30 | 10 |
| >50 IU | ≥8.5 | 36 | 12 |

The two algorithms used for patients in the BIF arms of the study utilize different fasting glucose (FG) targets and adjustment intervals: adjustment every 2 weeks targeting FG ≤140 mg/dL for algorithm 1, and adjustment every 4 weeks targeting FG ≤120 mg/dL for algorithm 2. The dose adjustments are set forth in Table 20 below:

**Table 20.**

| Criteria used to determine dose | Algorithm #1-Every 2 Weeks | Algorithm #2-Every 4 Weeks |
|---|---|---|
| < 70mg/dL | - 1.5 mg | - 1.5 mg |
| 71 to 99 mg/dL | - 1 mg | - 1 mg |
| 100 to 119 mg/dL | 0 mg | 0 mg |
| 120 to 139 mg/dL | 0 mg | + 0.5 mg |
| 140-180 mg/dL | + 1 mg | + 1 mg |
| > 180 mg/dL | + 1.5 mg | + 2 mg |
| Number of hypoglycemic episodes to trigger dose reduction | 1 | 1 |
| Dose reduced by | - 1 mg | - 1 mg |
| Dose adjustment interval | 14 days | 30 days |

Insulin degludec is titrated to FG target of ≤100mg/dL using a modified Riddle treat-to-target algorithm. Study participants (N=399) are randomized in a 1:1:1 ratio to 1 of 3 parallel treatment groups. The average age of participants is 60.2 years, baseline HbA1c was 8.1% and duration of diabetes 14.7 years. There are no statistically significant differences in demographics or baseline characteristics across the 3 treatment groups.

Both BIF groups achieve non-inferiority (non-inferiority margin = 0.4%) for the primary endpoint of HbA1c change from baseline to Week 32 with a mean±SE reduction for BIF algorithm 1, BIF algorithm 2 and insulin degludec of 0.6±0.1%, 0.6±0.1% and 0.7±0.1%, respectively. In line with the different plasma glucose targets, insulin degludec achieved greater glucose lowering from baseline as compared to the BIF arms. Similarly, both BIF dosing groups show significantly fewer hypoglycemic events compared to insulin degludec (all documented events as well as nocturnal events) when assessing events <70 mg/dL (3.9 mmol/L). Hypoglycemic events <54 mg/dL (3.0 mmol/L - all documented events as well as nocturnal events) were not significantly different between the three dosing groups. Both BIF groups had a statistically significantly smaller increase in body weight compared to insulin degludec from baseline to Week 32.

In summary, BIF, when administered weekly according to either dosing algorithm, was noninferior to insulin degludec for glycemic control as measured by change in HbA1c after 32 weeks with a lower rate of documented and nocturnal hypoglycemia <70 mg/dL and less weight gain. Additionally, no safety signals were detected.

The results are confirmatory of the predictive power of the PK/PD-IGI model and are used to update the model. The updated model is used to develop additional dose titration schemes studied in clinical trials described in more detail below.

### Patients Previously Treated with Multiple Daily Injection Therapy.

A multicenter, randomized, open-label, parallel, comparator-controlled study with 3 study periods is designed to evaluate the efficacy and safety of a dosing algorithm for BIF compared with insulin degludec in patients with T1DM treated with MDI without interruption for at least 3 months. The study will last 26 weeks to have an adequate duration of exposure necessary to assess efficacy and safety of insulin efsitora alfa. Insulin degludec is an unblinded active comparator in this study and will be used to compare the effects of BIF on glycemic control, hypoglycemia, and weight gain with a daily basal insulin.

Efficacy and safety assessments included in this study are generally regarded as reliable and accurate with respect to the efficacy and safety assessments in individuals and populations with T1DM. The primary efficacy measure is HbA1c change from baseline to Week 26. Secondary efficacy assessments for this study are: HbA1c change from baseline to Week 12; FG change from baseline to Weeks 12 and 26; Bolus insulin dose change from baseline to Weeks 12 and 26.

The following safety assessments will be evaluated as secondary objectives: Incidence and rate of hypoglycemia; Incidence of treatment-emergent SAEs; Clinical laboratory assessments with specific focus on liver aminotransferase changes.

Participants will be randomized in a 1:1 ratio to receive BIF subcutaneously weekly, dosed per the methods and regimens described herein, or insulin degludec subcutaneously daily, dosed per a modified Riddle algorithm.

The participants who complete the treatment will provide at least 80% statistical power to demonstrate noninferiority in the change in HbA1c from baseline to 26 weeks for BIF versus insulin degludec, based on the following assumptions:
- True mean difference = 0%
- SD of 1.1%
- NIM of 0.4%
- Using 2-sided alpha level of 0.1

All tests of treatment effects will be conducted at a 2-sided alpha level of 0.1, unless otherwise stated, and all confidence intervals (CIs) will be given at a 2-sided 90% level. The maximum total duration of study participation for each participant is up to 33 weeks, across the following study periods:
- Study Period 1: screening and lead-in period, approximately 2 weeks
- Study Period 2: treatment period, 26 weeks
- Study Period 3: safety follow-up period, 5 weeks

Procedures are implemented at screening to establish eligibility for inclusion in the study. Inclusion criteria are set forth in Table 21 below.

**Table 21.**

| |
|---|
| Type of Study Participant and Disease Characteristics |
| Have a diagnosis of T1DM for at least 1 year. A diagnosis of T1DM is based on medical history with a fasting C-peptide ≤0.30 nmol/L at screening or before randomization. |
| Have been using MDIs without interruption for at least 3 months prior to screening. |
| Study Participant Characteristics |
| Women of childbearing potential who abstain from sexual relationships with males or agree to use1 highly effective method of contraception or a combination of 2 effective methods of contraception. |
| Women not of childbearing potential. |
| All participants must meet the following criteria: |
| • Are at least 18 years of age, at the time of signing the informed consent. |
| • Have HbA1c values of 5.6% to 9.5% inclusive, as determined by the central laboratory at screening. |
| • Have been treated with a stable regimen of once- or twice-daily insulin glargine (U-100 or U-300), insulin detemir, or insulin degludec (U-100 or U-200) for 3 months prior to screening. |
| • Are currently treated with the same SC rapid-acting analog insulin (insulin lispro U-100 or U-200, insulin aspart, FiAsp, or insulin glulisine) in MDI for at least the last 30 days prior to screening. |
| • Have a body mass index (BMI) ≤35 kg/m², with no significant weight gain or loss in the past 3 months (≥5%). |
| • In the investigator's opinion, are well-motivated, capable, and willing to: learn how to self-inject treatment; maintain study diaries, as required for this protocol; wear study-provided CGM (Dexcom G6) during the complete study duration without interruption and use this device for therapeutic decision-making; must have a normal wake/sleep pattern such that midnight to 0600 hours will reliably reflect a usual sleeping period. |
| • In the investigator's opinion, are proficient in: counting carbohydrates; adjusting meal- and correction boluses based on glucose readings with a stable insulin/carbohydrate ratio as well as correction factors; and adjusting insulin and dietary therapy during special situations (e.g., exercise, stress, intermittent diseases) |
| • Are willing and able to follow the visit schedule during the complete duration of the trial. |
| • Capable of giving signed informed consent. |

Exclusion criteria are set forth in Table 22 below.

**Table 22.**

| |
|---|
| Medical Conditions |
| Have had more than 1 emergency room visit or hospitalization due to poor glucose control (hyperglycemia or diabetic ketoacidosis [DKA]) within 6 months prior to screening. |
| Have had any episodes of severe hypoglycemia (defined as requiring assistance due to neurologically disabling hypoglycemia) and/or hypoglycemia unawareness within the 6 months prior to screening. |
| Have significant lipohypertrophy, lipoatrophy, scars, or h/o abscess in areas of injection. |
| Have vision or hearing loss that impairs recognition of CGM screens, alerts and alarms. |
| Cardiovascular (CV): have had any of the follow CV conditions: acute myocardial infarction, New York Heart Association Class III or IV heart failure, or cerebrovascular accident (stroke) |
| Gastrointestinal: have gastroparesis or have undergone gastric bypass (bariatric) surgery or restrictive bariatric surgery (e.g., Lap-Band^{®}) prior to screening |
| Hepatic: have acute or chronic hepatitis, or obvious clinical signs or symptoms of any other liver disease except non-alcoholic fatty liver disease (NAFLD) (i.e., patients with NAFLD are eligible for participation), and/or have elevated liver enzyme measurements, as determined by the central laboratory at screening and as indicated below: |
| • Total bilirubin level (TBL) >2x the upper limit of normal (ULN), or |
| • Alanine aminotransferase (ALT)/serum glutamic pyruvic transaminase (SGPT) >2.5x ULN, or |
| • Aspartate aminotransferase (AST)/serum glutamic oxaloacetic transaminase (SGOT) >2.5x ULN |
| Renal: |
| • Have history of renal transplantation |
| • Are currently receiving renal dialysis |
| • Have serum creatinine >2.0 mg/dL (177 µmol/L) at screening, or |
| • Have an estimated glomerular filtration rate (eGFR) of <30 mL/min/1.73 m² |
| Have experienced significant weight loss or gain (>5%) in body weight in the 3 months prior to screening. |
| Have active or untreated malignancy, or have been in remission from clinically significant malignancy (other than basal cell or squamous cell skin cancer) for less than 5 years or are at increased risk for developing cancer or a recurrence of cancer in the opinion of the investigator. |
| Have known hypersensitivity or allergy to any of the study medications or their excipients. |
| Have any other serious disease or condition (e.g., known drug or alcohol abuse/regular consumption or psychiatric disorder) that, in the opinion of the investigator, would pose a significant risk to the patient, preclude the patient from following and completing the protocol, or interfere with the interpretation of safety, efficacy, or PD data. |
| Alcohol abuse/regular consumption is defined as an average daily intake of >3 units for males or >2 units for females within 6 months prior to the study. One unit is equivalent to 8 g of alcohol: a half-pint (~240 mL) of beer, 1 glass (125 mL) of wine or 1 (25 mL) measure of spirits. |
| Have had a blood transfusion or severe blood loss within 3 months prior to screening or have any hematologic condition that may interfere with HbA1c measurement (e.g., hemoglobinopathy, hemolytic anemia, sickle-cell disease). |
| Have fasting triglycerides >400 mg/dL or non-fasting triglycerides >600 mg/dL. |
| Women of childbearing potential who |
| i) are pregnant or intend to become pregnant |
| ii) are lactating/breastfeeding (including the use of a breast pump) |
| iii) are unwilling to remain abstinent or use birth control as described in Appendix 4 |
| iv)test positive for pregnancy at the time of screening (Visit 1). Note: a urine pregnancy test is conducted at Visit 3. |
| Prior/Concomitant Therapy |
| Are taking drugs that may significantly affect glycemic control (e.g., niacin [allowed if <1.0 g/day], bile acid sequestrants). |
| Are receiving chronic (lasting longer than 14 consecutive days) systemic glucocorticoid therapy (including intravenous, intramuscular, SC, and oral) or intra-articular (but excluding topical, intraocular, intranasal, and inhaled preparations), or have received such therapy within 4 weeks immediately prior to screening with the exception of replacement therapy for adrenal insufficiency. |
| Are currently taking or have taken within the 3 months preceding screening, prescription or over-the-counter medications to promote weight loss. Patients who participate must agree not to initiate a diet and/or exercise program during the study with the intent of reducing body weight other than the lifestyle and dietary measures for diabetes treatment. |
| Are taking total daily dose of insulin >100 I.U. at the time of screening. |
| Are receiving any oral or injectable medication intended for the treatment of diabetes mellitus other than rapid-acting and basal analog insulin in MDI in the 90 days prior to screening. |
| Are using or have used blood pressure-lowering medication at a dose that has not been stable for 1 month prior to screening. |
| Prior/Concurrent Clinical Trial Experience |
| Are currently enrolled in any other clinical study involving an IP or any other type of medical research judged not to be scientifically or medically compatible with this study. |
| Have participated, within the last 30 days in a clinical trial involving an IP. If the previous IP has a long half-life, 3 months or 5 half-lives (whichever is longer) should have passed. |
| Have previously completed or withdrawn from this study or any other study investigating BIF. |

Upon signing the informed consent form (ICF), at Visit 1 study participants will be trained on disease monitoring and disease management procedures, study diaries, and study procedures. Electronic participant diaries and participant paper note sheets will be dispensed at Visit 2 and as specified in the Schedule of Activities for future visits. Collection of baseline self-monitoring blood glucose (SMBG) profiles via continuous glucose monitoring (CGM) will start at Visit 2. Participants will continue on their same regimen of basal and short acting insulin during the lead-in period up to randomization. For all study participants meeting study entry criteria, the Dexcom G6^{®} CGM device will be inserted and activated at (Visit 2). Participants will record two 6-point glucose profiles (using the CGM device) at non-consecutive days between Visit 2 and Visit 3. The profile should include readings before and 2 hours after each major meal of the day (breakfast, lunch, and dinner). A standard system, Dexcom G6, will be used according to manufacturer's directions for CGM in an unblinded mode. The study participants will wear this device beginning at Visit 2. In addition, all study participants will be allowed to use their personal blood glucose (BG) meters for additional BG testing, or for taking SMBG measurements during the outpatient period. Therapeutic decisions will be based on the CGM readings. Blood glucose meters must not be used to calibrate the CGM device. CGM calibration must be done using the code provided with each sensor. Participants must use the study-specific CGM receiver and are not allowed to connect the transmitter of the CGM system to a personal smartphone to ensure data availability for download from the receiver at the respective visits.

At Visit 2, study participants who fulfill eligibility criteria will be trained on the use of the CGM device, CGM sensor replacement, interpretation of CGM-based BG values and alarms, and the requirements for CGM. For the first CGM session, study participants meeting all study-entry criteria will have the CGM sensor inserted as part of the Visit 2 activities.

Participants who continue to be eligible for the study will be randomized to 1 of the 2 treatment groups. Following randomization at Visit 3, participants will participate in a 26-week treatment period. For patients randomized to the BIF arm the following guidance for IP administration is applicable:
- During Weeks 0 to 8, site personnel will administer IP at the site. Participants will receive education and training on how to self-administer IP.
- During Weeks 9 to 12, IP will be reconstituted and administered at the site by the participant, under supervision of trained site personnel to assure that the participant is capable of self-administration.
- During Weeks 13 to 25, outside of titration visits IP can be self-administered by the participant at home unless local regulations require administration at the site, or optionally can be administered once weekly by site personnel.
- Information on self-injection can be reviewed as necessary throughout the study. Additional visits to ensure correct dosing, titration, and study drug administration can occur anytime during the study if deemed necessary by the investigator.

Following a once-weekly subcutaneous (SC) administration of BIF, the time to reach steady-state glucose level is estimated between 4 to 16 weeks based on the long half-life of BIF. As it may be desirable to achieve the therapeutic goal of BIF in less than 12 weeks with low risk of hyperglycemia, a loading dose strategy may be appropriate where a first dose sufficient to achieve an efficacious exposure is given initially, followed by individually optimized weekly dose adjustments to achieve target response. Thus, a dosing algorithm was developed specifically for BIF to rapidly achieve glycemic goals. The dosing algorithm described below will be used by investigators for initiating and adjusting BIF to achieve a target FG ≤100 mg/dL (<5.6 mmol/L).

Therefore, a single loading dose, followed by weekly dose adjustments is recommended. The loading dose is determined based on the patient's previously used basal insulin dose, baseline fasting glucose, and available BIF data to inform the 3x loading dose strategy. Dose adjustments are based on prior fasting glucose and hypoglycemia events. Alterations to the doses recommended by these dose adjustment algorithms are also under discretion of the investigator and will take hypoglycemia and other study participant safety concerns into account. In case of a deviation from the algorithm-recommended dose, a medical rationale for this deviation must be documented by the principal investigator.

The starting BIF dose is determined based on both the prior basal insulin dose and baseline fasting glucose. Participants may enter the study using insulin glargine, detemir, or degludec. Consistent with product labeling these insulins may be transitioned on a unit-for-unit basis and for the purposes of this protocol are considered equivalent. Because of the long half-life of BIF , the change in fasting glucose response may take several weeks to reach a steady-state response; therefore, to minimize the time needed to achieve the desired target glycemic response, a loading dose strategy will be used for the first dose only.

Determination of the starting dose, i.e., the loading dose, will be conducted according to the following directions for converting current basal insulin dose (in Units, U) to the dose of BIF (in mg).
1. Obtain the prior daily basal insulin dose of insulin glargine, detemir, or degludec assessed during the lead-in period. Calculate the "basal insulin equivalent dose" of BIF (in mg) by dividing the total daily dose of basal insulin (U) by the conversion factor of 7 U/mg.
2. Adjust the dose of BIF calculated in step 1 above according to median baseline fasting glucose and the participant's prior basal insulin dose (U) category as shown in Table 23 below. This is the expected starting weekly dose.

| **Median Baseline FG (mg/dL)** | **Median Baseline FG^{a} (mmol/L)** | **BIF Basal Insulin Equivalent Dose Adjustment (mg)^{b}** | | |
|---|---|---|---|---|
| | | **Prior Basal Insulin Dose ≤ 15 U** | **Prior Basal Insulin Dose 16 - 30 U** | **Prior Basal Insulin Dose > 30 U** |
| < 80 | < 4.4 | - 0.25 | - 1 | - 1.5 |
| 80 - 100 | 4.4 - 5.5 | No change | No change | No change |
| 101 - 140 | 5.6 - 7.7 | + 0.25 | + 0.5 | + 0.75 |
| 141 - 180 | 7.8 - 10.0 | + 0.5 | + 1 | + 1.5 |
| 181 - 220 | 10.1 - 12.2 | + 0.75 | + 1.5 | +2 |
| >220 | >12.2 | + 1 | +2 | +3 |

| | | | | |
|---|---|---|---|---|
| Table 23. BIF Dose Adjustment (mg). ^{a} Conversions from mg/dL to mmol/L were rounded off to prevent overlap between threshold ranges and to address that the glucose meter displays 1 significant digit after the decimal when reporting mmol/L readings. ^{b} This dose adjustment is added to the Basal Insulin Equivalent Dose to obtain the total weekly dose of BIF in mg. | | | | |

3. Multiply the expected starting weekly dose obtained in step 2 above by 3 to obtain the loading dose.

For example, if a participant uses 35 U of degludec each day, that dose is converted to 5 mg of BIF according to step 1. If that participant had FG value of 155 mg/dL, the dose adjustment for this participant according to step 2 and Table 15 would be +1.5 mg. Thus, the participant's expected starting weekly dose of BIF would be 6.5 mg, and the participant's loading dose, according to step 3, would be 19.5 mg. At the time of each subsequent site visit, the investigator will assess the study participant's glycemic control for the previous week and, if necessary, inform the study participant about any needed dose adjustment. Dose adjustments will be determined based on the median of the FG (determined from SMBG obtained from the CGM system, referred to as SMBG below) of at least 3 days (up to 7 days) obtained in the previous week leading up to the visit.

In order to enable achievement of target FG of ≤100 mg/dL (<5.6 mmol/L) quickly while minimizing risk of hyperglycemia, a dose adjustment strategy has been designed that provides for relatively larger adjustments for the first weekly dose following the loading dose (i.e., visit 4 in the present study) than the second weekly dose following the loading dose (i.e., visit 5 in the present study), and relatively larger adjustments for the second weekly dose following the loading dose (i.e., visit 5 in the present study) than for all subsequent weekly doses. This dose adjustment strategy will be executed according to the following instructions:
1. Obtain the starting total weekly dose as described above. (This is 1/3^{rd} the loading dose administered).
2. Use Table 24 below to determine the dose adjustment needed after carefully reviewing the participant's fasting glucose (3-7 days prior to visit) and hypoglycemia documented since the last BIF dose.

**Table 24. Weekly Dose Adjustment. ^{a} Based on median FG from at least 3 fasting glucose readings from previous week. ^{b} Conversions from mg/dL to mmol/L were rounded off to prevent overlap between threshold ranges and to address that the glucose meter displays 1 significant digit after the decimal when reporting mmol/L readings. ^{c}D for Week 1 (Visit 4) is 1/3 of the dose administered at (Week 0, Visit 3). ^{d} D for Week 2 (Visit 5) is the dose administered at Week 1 (Visit 4). ^{e} D for Subsequent Visits is the dose administered one week prior to the current visit. ^{f} The BIF dose may not be increased if any SMBG reading was documented at <70 mg/dL (<3.9 mmol/L) at any time in the preceding week. If multiple episodes of hypoglycemia with SMBG <70 mg/dL (<3.9 mmol/L) were recorded, follow guidance for dose reduction according to the applicable Visit number. Dose decreases of 1 mg should be made when severe hypoglycemia (requiring assistance) occurred, or if any SMBG was documented at ≤54 mg/dL (≤3.0 mmol/L) in the preceding week.**

| **Median Fasting glucose (mg/dL)^{a}** | **Median Fasting glucose (mmol/L)^{a,b}** | **Week 1 (Visit 4) (2^{nd} dose)^{c}** | **Week 2 (Visit 5) (3^{rd} dose)^{d}** | **Week 3 (Visit 6) and all Subsequent Weeks^{e}** |
|---|---|---|---|---|
| <80 mg/dL or have any nocturnal hypoglycemia or multiple (≥3) episodes of hypoglycemia^{f} | <4.4 mmol/L or have any nocturnal hypoglycemia or multiple (≥3) episodes of hypoglycemia^{f} | D^{c} - 1.5 mg | D^{d} - 1 mg | D^{e} - 0.5 mg |
| 80 - 100 mg/dL | 4.4 - 5.5 | D^{c} - 1 mg | D^{d} - 0.5 mg | D^{e} |
| 101 - 140 mg/dL | 5.6 - 7.7 | D^{c} | D^{d} | D^{e} + 0.25mg |
| 141 - 180 mg/dL | 7.8 - 10.0 | D^{c} + 1 mg | D^{d} + 0.5 mg | D^{e} + 0.25mg |
| > 180 mg/dL | >10.0 | D^{c} + 2 mg | D^{d} + 1 mg | D^{e} + 0.5 mg |

For example, for the participant with a loading dose of 19.5 mg described above, the starting weekly dose was 6.5 mg. If that participant reported no hypoglycemia since the 19.5 mg loading dose was administered and had a median FG of 150 mg/dL, using the Week 1 (Visit 4) column, the weekly dose should be increased by 1 mg. Thus, the participant's first weekly dose following the loading dose (i.e., visit 4) would be 7.5 mg. For the next weekly dose (i.e., visit 5), if the participant reported no hypoglycemia since the 7.5 mg dose and had a median FG of 153 mg/dL, using the Week 2 (Visit 5) column, the weekly dose should be increased by 0.5 mg. Thus, the next weekly dose would be 8 mg. For the next weekly dose, using the "Week 3 (Visit 6) and all subsequent weeks" column, if the participant had a median FG of 133 mg/dL but reported one episode of nocturnal hypoglycemia since the last dose and, the dose should be reduced by 0.5 mg. Thus, the next weekly dose would be 7.5 mg. Subsequent visits are managed using the same approach as for Visit 6 (Week 3), always confirming the BIF dose administered at the prior visit, the median fasting glucose during the week prior to the visits, and hypoglycemia status since the last dose of study drug.

Adherence to the dosing algorithm is required from Visit 3 (randomization) up to Week 25 and will be monitored periodically by the study team. Safety of study participants will be closely monitored during the early stages of dose titration to determine whether adjustments to the conversion and dose adjustment algorithms are needed. In situations where study participant safety is a concern, investigators may make adjustments to the dose recommended by the dose-adjustment algorithm in Table 8.

For patients randomized to the insulin degludec arm insulin degludec will be self-administered daily by participants after a training and first administration under site personnel supervision on Day 1. The starting dose for insulin degludec is the same dose as the basal insulin that the study participant used prior to entering the study. For subsequent dose adjustments, a dosing algorithm (adapted from Riddle et al. 2003) will be used by investigators for initiating and adjusting insulin degludec to target an FG ≤100 mg/dL (<5.6 mmol/L) for patients to achieve glycemic goal.

In situations where patient safety is a concern or where dose adjustments have not had the desired therapeutic effect, investigators may make increased or decreased adjustments to the dose of insulin degludec recommended by the dose-adjustment algorithm and have to document the clinical rationale for the deviation as well as inform Lilly Medical per email.

At the time of site visit or telephone visit, the investigator will assess the patient's glycemic control for the previous week and, if necessary, inform the patient about any needed dose adjustment. The dose will be based on the patient's blood glucose value. Adherence to the dosing algorithm provided for this study is mandatory from Visit 3 (randomization) up to Visit 20 (Week 26), inclusive. Dose increases may be made at weekly intervals and no sooner than 5 days following the last dose increase. In some cases, patient visits could occur sooner than 5 days apart due to the allowable visit window; however, there should be no dose increase if at least 5 days have not elapsed since the previous dose increase. In contrast, the insulin degludec dose may be reduced at any time on the judgment of the investigator. Any deviations from this guidance must be supported by clinical evidence and accompanied by information of Lilly Medical. Insulin dose algorithms have been reviewed by Strange (2007). The insulin degludec dose increase algorithm is adapted from Riddle et al. 2003 and will be determined based on the definition of what is termed the "Algorithm FG." The Algorithm FG is the median of the FG (determined from SMBG) of Days 5, 6, and 7 since the last insulin dose increment. If the interval since the last dose adjustment has been longer than 7 days, the dose increase is determined by the median of the FG of the last 3 days. If the patient only measured their FG on 2 of the last 3 days, then the lesser of those 2 FG values should be used as the Algorithm FG. If only 1 FG measurement is available for the last 3 days, then the investigator should use his/her discretion in determining whether there should be a dose adjustment based on that single FG value.

As it is desired to achieve glycemic goals in as many patients as possible, contingencies are provided to accommodate prespecified protocol visits and to increase the dose of insulin degludec in a timely manner. Therefore, if the last dose increment was 5 days previous, then the dose increment is determined by the Algorithm FG, which is the lesser of the 2 FG values from Day 4 and Day 5. If the dose increment was 6 days previous, then the Algorithm FG is the lesser of the 2 FG values from Day 5 and Day 6. In both of these cases, if only 1 of the 2 FG values is available, then the investigator should use his/her discretion in determining whether there should be a dose adjustment based on that single FG value.

The dose increase algorithm is defined as follows:
- If the Algorithm FG is 101 to 120 mg/dL (5.6 to 6.7 mmol/L), increase the dose by 2 U;
- If the Algorithm FG is 121 to 140 mg/dL (6.8 to 7.8 mmol/L), increase the dose by 4 U;
- If the Algorithm FG is 141 to 180 mg/dL (7.9 to 10.0 mmol/L), increase the dose by 6 U; and
- If the Algorithm FG is >180 mg/dL (10.0 mmol/L), increase the dose by 8 U.

The treat-to-target FG is ≤100 mg/dL (<5.6 mmol/L). The insulin degludec dose will not be increased if any SMBG was documented at <70 mg/dL (<3.9 mmol/L) at any time in the preceding week. Dose decreases of 2 to 4 U per adjustment may be permitted if multiple episodes of hypoglycemia with SMBG <70 mg/dL (<3.9 mmol/L) were recorded, if severe hypoglycemia (requiring assistance) occurred, or if any SMBG was documented at ≤54 mg/dL (≤3.0 mmol/L) in the preceding week.

Participants in all arms will continue to use concomitant short-acting insulin throughout the treatment period. Discontinuation or changes to regimen are not permitted, except in situations where dose adjustment is required for medical reasons or when allowed per study protocol.

Study participants will be instructed to document fasting glucose (FG) each day in their electronic diary (eDiary) by using the values displayed on their CGM device after wakening. In addition, two 6-point SMBG profiles (prior to and 2 hours after the morning, midday, and evening meals) should be done on nonconsecutive days in the week prior to the required visits.

Participants who develop severe, persistent hyperglycemia will receive an unscheduled intensification of their insulin treatment based on clinical judgment of the investigator. Participants who need unscheduled intensification of their insulin treatment will continue on IP in the trial until they complete all study visits.

All randomized participants should have a comprehensive efficacy and safety evaluation approximately 1 week after the last dose of BIF and 1 day after the last dose of insulin degludec, and a safety follow-up visit approximately 6 weeks after the last dose of BIF and 5 weeks after the last dose of insulin degludec. During the safety follow-up study period, participants will continue CGM and will be switched back to their previously used basal insulin therapy if appropriate.

Participants who discontinue IP prior to the completion of the treatment period will be encouraged to remain in the study and to complete any scheduled study procedures that occur until Visit 20. Participants remaining in the study will receive an appropriate glucose-lowering regimen.

Participants who discontinue IP for any reason and are unwilling to return for a safety follow-up visit, will be asked to perform an early termination (ET) visit as their final study visit.

Results from modeling simulations of the titration scheme tested support noninferiority in the change in HbA1c from baseline for BIF versus insulin degludec.

### Insulin-Naïve Patients.

A multicenter, randomized, open-label, parallel, comparator-controlled study with 3 study periods is designed to evaluate the efficacy and safety of BIF compared with insulin degludec in patients with T2DM treated with a stable dose of metformin (alone or in combination with a stable dose of a DPPIV inhibitor and/or a SGLT2 inhibitor) for at least 3 months prior to screening. The study design is largely the same as the Phase 2 study in patients with T1D described above, but with a different target patient population and some other corresponding differences.

The population for this study includes patients who: have a diagnosis of T2DM treated with a stable dose of metformin (alone or in combination with a stable dose of a DPPIV inhibitor and/or a SGLT2 inhibitor) for at least 3 months prior to screening; are 18 to 75 years of age; have a baseline HbA_{1c} value of 7.0% to 9.5%, inclusive, at screening; have been treated with a stable dose of metformin (alone or in combination with a stable dose of a DPPIV inhibitor and/or a SGLT2 inhibitor) for 3 months prior to screening and be willing to continue stable dosing throughout the study; and have a body mass index (BMI) between 20 and 45 kg/m², inclusive, with no significant weight gain or loss in the past 3 months (≥5%).

The population excludes participants who: have history of greater than 1 episode of ketoacidosis or hyperosmolar state/coma requiring hospitalization in the 6 months prior to screening; have had any episodes of severe hypoglycemia and/or hypoglycemia unawareness within the 6 months prior to screening; have had any of the follow CV conditions: acute myocardial infarction, New York Heart Association Class III or IV heart failure, or cerebrovascular accident (stroke); have undergone gastric bypass (bariatric) surgery or restrictive bariatric surgery (e.g., Lap-Band^{®}) prior to screening; have acute or chronic hepatitis, or obvious clinical signs or symptoms of any other liver disease except non-alcoholic fatty liver disease (NAFLD) (i.e., patients with NAFLD are eligible for participation), and/or have elevated liver enzyme measurements, as determined by total bilirubin >1.5x the upper limit of normal (ULN) in the absence of Gilbert's syndrome, Alanine aminotransferase (ALT)/serum glutamic pyruvic transaminase (SGPT) >2.0x ULN, or Aspartate aminotransferase (AST)/serum glutamic oxaloacetic transaminase (SGOT) >2.0x ULN; have an estimated glomerular filtration rate (eGFR) <30 mL/min/1.73 m²; have fasting triglycerides >400 mg/dL or non-fasting >600 mg/dL; have experienced significant weight loss or gain (>5%) in body weight in the 3 months prior to screening (Visit 1); have active or untreated malignancy, or have been in remission from clinically significant malignancy (other than basal cell or squamous cell skin cancer) for less than 5 years or are at increased risk for developing cancer or a recurrence of cancer in the opinion of the investigator; have known hypersensitivity or allergy to any of the study medications or their excipients; have any other serious disease or condition (for example, known drug or alcohol abuse/regular consumption or psychiatric disorder) that, in the opinion of the investigator, would pose a significant risk to the patient, preclude the patient from following and completing the protocol, or interfere with the interpretation of safety, efficacy, or PD data; have had a blood transfusion or severe blood loss within 3 months prior to screening or have any hematologic condition that may interfere with HbA1c measurement (e.g., hemoglobinopathy, hemolytic anemia, sickle-cell disease); are taking drugs that may significantly affect glycemic control (e.g., niacin [allowed if <1.0 g/day], bile acid sequestrants or pentoxyphylline); are receiving chronic (>14 days) systemic glucocorticoid therapy (excluding topical, intraocular, intranasal, or inhaled preparations) or have received such therapy for >14 days within the month preceding screening; are currently taking or have taken within the 3 months preceding screening, prescription or over-the-counter (OTC) medications to promote weight loss. Patients who participate must agree not to initiate a diet and/or exercise program during the study with the intent of reducing body weight other than the lifestyle and dietary measures for diabetes treatment; have been treated with a sulfonylurea (SU), a glitazone, alpha-glucosidase inhibitor, a GLP-1 receptor agonist or insulin in the 3 months prior to screening with the exception of short-term use of insulin for acute conditions (≤14 days within the last 6 months prior to screening); are using or have used blood pressure-lowering medication at a dose that has not been stable for 1 month prior to screening; and women of childbearing potential who are pregnant or intend to become pregnant, are lactating/breastfeeding (including the use of a breast pump) or are unwilling to remain abstinent or use birth control.

Participants on metformin (alone or in combination with a stable dose of a DPPIV inhibitor and/or a SGLT2 inhibitor) will continue on their same dose to allow reliable assessment of HbA1c at randomization. If participants develop a condition that is a contraindication for the use of metformin, a DPPIV inhibitor and/or a SGLT2 inhibitor (if used at screening) or initiate other agents that are prohibited between screening and randomization, they will be considered ineligible and will be discontinued from the trial before randomization.

For all participants meeting study entry criteria except for reporting of screening clinical laboratory assessments, a LibrePro sensor for flash glucose monitoring (FGM) will be inserted and activated at Visit 1 for baseline FGM assessment. If the patient is ultimately determined a screen failure, the sensor can be removed and discarded or the patient can continue wearing for up to 14 days and return to the site for reading and diagnostics. All confirmed eligible participants should continue wearing the sensor for up to 14 days and to wear the sensor to Visit 3 for removal or bring the sensor with them to Visit 3 if this visit occurs longer than 14 days after insertion.

Participants will undergo 3 FGM sessions using the LibrePro System (Abbott) in the 14-day period prior to randomization, Visit 15, and Visit 20. Patients will remain blinded to these assessments until the study is completed.

The (Week 0, Visit 3) BIF loading dose is based on the baseline median fasting glucose and body weight at Visit 3. Like the study in T1D patients described above, a loading dose strategy is employed to minimize the time needed to achieve the desired target glycemic response and to achieve steady-state concentration within the first week. The loading dose comprises a single dose that is increased approximately 3-fold from the expected starting weekly maintenance dose based on the patient's median fasting glucose and body weight at the start of the study, and is determined using Table 25 below.

**Table 25. Determination of Loading Dose of BIF at Visit 3 using Median of Baseline Fasting Glucose and Body Weight. ^{a} Conversions from mg/dL to mmol/L were rounded off to prevent overlap between threshold ranges and to address that the glucose meter displays 1 significant digit after the decimal when reporting mmol/L readings.**

| **Median Baseline FG (mg/dL)** | **Median Baseline FG^{a} (mmol/L)** | **Body Weight (kg) at Visit 3** | | | |
|---|---|---|---|---|---|
| | | **≤ 80** | **80.1 - 100** | **100.1 - 120** | **≥ 120.1** |
| ≤ 140 | ≤ 7.7 | 3 mg | 4.5 mg | 6 mg | 7.5 mg |
| 141 - 180 | 7.8 - 10.0 | 6 mg | 7.5 mg | 10.5 mg | 12 mg |
| 181 - 220 | 10.1 - 12.2 | 9 mg | 10.5 mg | 12 mg | 13.5 mg |
| > 220 | > 12.2 | 12 mg | 13.5 mg | 15 mg | 16.5 mg |

For example, if a participant weights 83 kg and has a median FG of 185 mg/dl, the initial dose of BIF is 10.5 mg. This 10.5 mg dose represents a 3-fold increase in the weekly maintenance dose expected to be needed based on the patient's median fasting glucose and body weight at the start of the study, i.e., based on the participant's weight and FG his weekly maintenance dose would be expected to be 3.5 mg. In order to reach target FG values quickly, however, the actual weekly maintenance dose to be administered the week following the loading dose is adjusted based on the participants FG values and any incidents of hypoglycemia during the subsequent week, as described in more detail below.

The treat-to-target FG is ≤100 mg/dL (<5.6 mmol/L). Dose adjustments for subsequent weekly maintenance doses of BIF will be determined based on the median of the FG of at least 3 days (up to 7 days) obtained in the previous week leading up to the visit according to Table 26 below. All doses should be rounded to the nearest 0.25 mg.

**Table 26. Weekly Dose Adjustment of BIF using the Previous Week's dose (D),**

| **Median FG (mg/dL)^{a}** | **Median FG (mmol/L)^{a,e}** | **Week 1 (Visit 4) (2^{nd} dose)** | **Week 2 (Visit 5) (3^{rd} dose)** | **Subsequent Week^{c}** | |
|---|---|---|---|---|---|
| | | | | **Dose ≤5 mg** | **Dose >5 mg** |
| < 80 mg/dL or have any nocturnal hypoglycemia or multiple episodes of hypoglycemia^{d} | < 4.4 mmol/L or have any nocturnal hypoglycemia or multiple episodes of hypoglycemia | D^{b} - 1.5 mg | D^{b} - 1.5 mg | D^{c} - 1.5 mg | D^{c} - 2 mg |
| 80 - 100 mg/dL | 4.4 - 5.5 | D^{b} - 1 mg | D^{b} - 0.5 mg | No change | No change |
| 101 - 140 mg/dL | 5.6 - 7.7 | No change | No change | D^{c} + 0.25 mg | D^{c} + 0.5 mg |
| 141 - 180 mg/dL | 7.8 - 10.0 | D^{b} +3 mg | D^{b} + 1.5 mg | D^{c} + 0.5 mg | D^{c} + 1 mg |
| > 180 mg/dL | > 10.0 | D^{b} + 5 mg | D^{b} + 3 mg | D^{c} + 0.75 mg | D^{c} + 1.5 mg |

Median Fasting Glucose and Hypoglycemic Episodes. ^{a} Based on median fasting glucose from at least 3 fasting glucose readings from previous week. ^{b} D for Week 1 (Visit 4) is 1/3 of the initial dose from Table 1. ^{d} for Week 2 (Visit 5) is Week 1 (Visit 4) dose. ^{c} BIF dose adjustment starting at Week 3 (Visit 6) will depend on whether previous (Week 2) dose (D) is ≤5 mg or >5 mg and will be adjusted weekly up to Week 12 (Visit 15), then at Week 16 (Visit 17), Week 20 (Visit 18), and Week 24 (Visit 19). D The BIF dose may not be increased if any SMBG reading was documented at <70 mg/dL (<3.9 mmol/L) at any time in the preceding week. If multiple episodes of hypoglycemia with SMBG <70 mg/dL (<3.9 mmol/L) were recorded, follow guidance for dose reduction according to the applicable Visit number. Dose decreases of 1 mg should be made when severe hypoglycemia (requiring assistance) occurred, or if any SMBG was documented at ≤54 mg/dL (≤3.0 mmol/L) in the preceding week. ^{e} Conversions from mg/dL to mmol/L were rounded off to prevent overlap between threshold ranges and to address that the glucose meter displays 1 significant digit after the decimal when reporting mmol/L readings.

For example, for the participant described above who received a loading dose of 10.5 mg, the expected starting weekly dose would have been 3.5 mg. During the subsequent week, the participant reported no hypoglycemia and had a median FG of 175 mg/dL. Using the week 1 (Visit 4) column of Table 18, the participant's first weekly maintenance dose following the loading dose would be 6.5 mg. During the week following administration of the first weekly maintenance dose, the participant reported no hypoglycemia and had a median FG of 163. Using the Week 2 (Visit 5) column, the weekly dose should be increased by 1.5 mg, so the second weekly maintenance dose would be 8.0 mg. During the week following administration of the second weekly maintenance dose, the participant had a median FG of 133 mg/dL. Using the "Subsequent Week" column, the weekly maintenance dose should be increased by 0.5 mg, so the third weekly maintenance dose would be 8.5 mg. Dose adjustments for all subsequent weeks are determined using the "Subsequent week" column, as described above for the third weekly maintenance dose.

An additional therapeutic intervention should be considered in patients who develop severe, persistent hyperglycemia after randomization based on the following criteria (FDA 2008):
a) average FG >270 mg/dL (>15.0 mmol/L) over any 2-week period or longer during the first 6 weeks post randomization; or
b) average FG >240 mg/dL (>13.3 mmol/L) over any 2-week period or longer from Week 6 to Week 12 post randomization; or
c) average FG >200 mg/dL (>11.1 mmol/L) over any 2-week period or longer after Week 12.

Investigators should first confirm that the patient does not have an acute condition causing severe hyperglycemia and, after the first 12 weeks of the study, that the patient is fully compliant with the assigned therapeutic regimen. The investigator will decide, in consultation with the patient, on an appropriate glucose-lowering intervention (rescue intervention) after considering relevant clinical criteria. See table in Section 6.5 for allowed medications. Patients who receive a new intervention for hyperglycemia management should also continue administering IP for the remaining period in the trial.

Insulin degludec will be administered at a starting dose of 10 IU/day, as described in the approved product labeling. Subsequent dose adjustments are determined and administered as described above for the Phase 2 study in T1D patients.

Results from modeling simulations of the titration scheme tested support noninferiority in the change in HbA1c from baseline for BIF versus insulin degludec.

### Phase 3 Studies.

Multicenter, randomized, parallel, comparator-controlled, treat-to-target, with 3 study periods are designed that include screening/lead-in, treatment, and safety follow-up periods. The planned treatment periods are 26, 52, and 78 weeks. Patient populations include: patients with T2DM where were previously treated with a daily basal insulin (but not MDI); insulin naïve patients with T2DM; patients with T2DM being treated with MDI and patients with T1DM.

### T2DM patients being treated with a daily basal insulin (but not MDI)

A Phase 3, multicenter, randomized, open-label, comparator-controlled study to evaluate the efficacy and safety of BIF compared to insulin degludec in participants with T2D being treated with basal insulin. The study consists of a 3-week screening/lead-in period, a 78-week treatment period, and a 5-week safety follow-up period. The primary outcome is the change from baseline in HbA1c at Week 26.

Participants are eligible to be included in the study only if they meet preestablished inclusion criteria, including the following: 1. Are at least 18 years of age at screening (or older per local regulations); 2. Have a diagnosis of T2D according to the WHO criteria currently treated with basal insulin; 4. Have HbA1c value 6.5%-10% inclusive, at screening; 5. Have been treated with a stable regimen in the opinion of the investigator of one of the following insulin regimens used according to local product label with or without non-insulin diabetes therapy (up to 3 of the following: dipeptidyl peptidase (DPP-4) IV inhibitors; SGLT2 inhibitors; metformin; alpha-glucosidase inhibitors, GLP-1 receptor agonists) for at least 90 days prior to screening: once-daily U100 or U200 insulin degludec; once-daily U100 or U300 insulin glargine; once or twice-daily U100 insulin detemir, or; once or twice-daily human insulin NPH; 6. Have a body mass index less than or equal to 45 kg/m2, at screening.

Participants are excluded from the study if any of the following criteria applies: 1. Have had a significant weight gain or loss in the past 3 months in the investigator's opinion (for example, ≥5%); 2. Have a diagnosis of T1D, latent autoimmune diabetes, or specific type of diabetes other than T2D (for example, monogenic diabetes, diseases of the exocrine pancreas, drug-induced or chemical-induced diabetes); 3. Are currently receiving or have received any time in the past 6 months, any of the following insulin therapies (outside of pregnancy) prior to screening, except for short-term treatment of acute conditions, and up to a maximum of 4 continuous weeks: prandial insulin, insulin mixtures, inhaled insulin, U-500 insulin, or continuous subcutaneous insulin infusion therapy; 4. Have received any of the following nonallowed diabetes medications within 90 days prior to screening: glinides, sulfonylureas, pramlintide, or thiazolidinediones; 5. Have a history of greater than 1 episode of ketoacidosis or hyperosmolar state/coma requiring hospitalization in the 6 months prior to screening; 5. Are receiving chronic (>14 days) systemic glucocorticoid therapy (excluding replacement therapy for adrenal insufficiency; topical, intraocular, intranasal, or inhaled preparations or intra-articular injection) or have received such therapy for >14 days within the month preceding screening; 6. Cardiovascular: have had New York Heart Association Class IV heart failure or any of the following CV conditions in the past 3 months prior to screening: acute myocardial infarction, cerebrovascular accident (stroke), or coronary bypass surgery; 7. Gastrointestinal: have undergone gastric bypass (bariatric) surgery, restrictive bariatric surgery (for example, Lap-Band^{®}), or sleeve gastrectomy within 1 year prior to screening; 8. Hepatic: have acute or chronic hepatitis, cirrhosis, or obvious clinical signs or symptoms of any other liver disease, except NAFLD (that is, study participants with NAFLD are eligible for participation), and/or have elevated liver enzyme measurements, as determined by the central laboratory at screening and as indicated below: total bilirubin >2x the ULN; ALT/serum glutamic pyruvic transaminase >2.5x ULN; AST/serum glutamic oxaloacetic transaminase > 2.5x ULN, or ALP > 2.5x ULN ; 9. Renal: have a history of renal transplantation, are currently receiving renal dialysis, or have an eGFR <20 mL/min/1.73 m2, calculated by the CKD-EPI equation, as determined by the central laboratory at screening; 10. Have active or untreated malignancy, have been in remission from clinically significant malignancy (other than basal cell or squamous cell skin cancer) for less than 5 years, or are at increased risk for developing cancer or a recurrence of cancer in the opinion of the investigator; 10. Have known hypersensitivity or allergy to any of the study medications or their excipients; 11. Have any other serious disease or condition (for example, known drug or alcohol abuse or psychiatric disorder) that, in the opinion of the investigator, would pose a significant risk to the study participant, preclude the study participant from following and completing the protocol; 11. Have evidence of a current or recent, within 6 months' time frame, history of any substance use disorder(s) of any severity as defined by the Diagnostic and Statistical Manual of Mental Disorders-5 (DSM-5) in the opinion of the investigator, except disorders of nicotine or caffeine use; 12. Hematologic: have had a blood transfusion or severe blood loss within 90 days prior to Visit 1 (Week -3) or have known hemoglobinopathy, hemolytic anemia or sickle cell anemia, or any other traits of hemoglobin abnormalities known to interfere with the measurement of HbA1c in the opinion of the investigator.

Participants who meet entry criteria will be randomly assigned in a 2:1 (BIF:insulin degludec) ratio to the following treatment groups: BIF: once-weekly administration, and Insulin degludec: once-daily administration. The total duration of study participation for each participant, including screening and the posttreatment follow-up periods, is approximately 86 weeks, across the following study periods: Study Period I: Screening and Lead-In Period, 3 weeks; Study Period II: Treatment Period, 78 weeks, and; Study Period III: Safety Follow-Up Period, 5 weeks.

BIF will be administered once-weekly at approximately the same time and day each week using an insulin-prefilled pen (with 5-unit increments). If a dose is missed, it should be administered as soon as possible if at least 3 days (72 h) remain until the next scheduled dose. If an interval of less than 3 days remains before the next scheduled dose, the missed dose should be skipped and the next dose should be administered on the regularly scheduled day. In each case, participants can then resume their regular once-weekly dosing schedule. The day of weekly administration can be changed, if necessary, as long as the last dose has been administered at least 3 days earlier.

Insulin degludec (U100) will be administered daily at approximately the same time each day using commercially available insulin-prefilled pens (with 1-unit increments).

For BIF, the maximum number of units per injection in the BIF-prefilled pen is 400 units. Doses greater than 400 units a week (approximately 57 units per day of basal insulin [57 × 7 days = 399]) will require more than 1 injection. The maximum one-time loading dose for this study is 1600 units, and the maximum weekly dose is 1400 units.

For insulin degludec, the maximum number of units per injection of insulin degludec (U100) is 80 units. For participants who require >80 units per day, more than 1 injection will be required to administer the full daily dose of insulin degludec.

For both treatments, participants will be instructed to rotate injection sites from one injection to the next, even when injecting within the same region.

The participants' initial and weekly dose of BIF or daily dose of insulin degludec will be determined based on the participant's usual daily basal insulin dose prior to randomization. The starting doses take into consideration the prestudy basal insulin's label with regards to ability to do unit-to-unit conversions across insulin types/regimens. For participants whose prestudy basal insulin regimens are typically adjusted to the more conventional once-daily (U-100) basal insulin regimens (for example, twice-daily NPH or once-daily U-300 glargine), the total daily dose is reduced by 20% before multiplying by 7 to obtain the weekly dose equivalent.

The dosing and titration algorithms to be used will be based on the participant's treatment assignment to either BIF or insulin degludec as described in more detail below. Participants will be instructed to not take their prestudy basal insulin on the day of randomization since they will be dosed at the site with the allocated study insulin.

In both treatment groups, insulin adjustments will be made to achieve and maintain an FBG target of 80-120 mg/dL throughout the study. Active titration will occur in both treatment groups between Visits 3 to 15 (Weeks 0 to 12) which will be designated as the Titration Period with the goal to achieve insulin dose stabilization by Visit 15 (Week 12) with monthly adjustments during the Maintenance Period from Visit 15 to 38 (Weeks 12 to 78).

Participants in both treatment groups will perform self-monitored FBGs daily. A minimum of 3 FBG readings on separate days each week must be obtained so that investigators can review the data to make dose adjustments using the titration algorithms for once-weekly or once-daily insulins. The median FBG to use in determining the dose adjustments starting at Visit 6 (Week 3) is obtained from the 3 most recent FBGs in the previous week. The median is the middle value when the 3 values are placed in ascending or descending order. For example, if a participant's FBGs in the past 3 consecutive days were 180, 202, and 190, the median is 190. If the participant has only 2 FBG readings for the week, then the lesser of the readings should be used to determine the dose adjustments. If only 1 FBG measurement is available, determination if dose is changed using only a single FBG value is at the discretion of the investigator.

When considering any dose adjustments (in either treatment group), the investigator will review whether the participant experienced any documented episodes of hypoglycemia. If the participant experienced any documented BG ≤70 mg/dL (≤3.9 mmol/L) in the previous week, the dose should not be increased. Additionally, if criteria for Hypoglycemia Dose Reduction are met, the prior week's basal dose is decreased as described in more detail below.

In this section, dosing instructions for BIF are provided for dose initiation at Visit 3 (Week 0), and administration of the daily dose at Visit 4 (Week 1) and subsequent dosing/dose adjustments throughout the treatment period.

At the randomization visit (Week 0) the initiation dose is determined based on FBG from the week prior to randomization. Instructions in Table 27 should be followed for participants with FBG > 120 mg/dL and instructions in Table 20 for participants with FBG ≤ 120 mg/dL. Note: If a participant is on NPH twice-daily or U300 glargine, the prestudy basal insulin dose should be reduced by 20% when determining Usual Daily Dose of the prestudy basal insulin before calculating the Starting Weekly Dose.

**Table 27. BIF Loading Dose Calculation - Participants with FBG > 120 mg/dL.**

| Step | Action | Example Participant A (FBG > 120 mg/dL) |
|---|---|---|
| 1. | Determine usual prior daily basal dose from lead-in period | 30 units/day |
| 2. | Calculate Starting Weekly Dose (Usual Daily Dose multiplied by 7 and round to nearest 10) | 210 units/Week |
| 3. | Multiply Starting Weekly Dose by 3 to determine the Loading Dose | 630 units Loading Dose |

The Loading Dose should not exceed a dose of 1600 units. If the calculated Loading Dose is >1600 units, the maximum loading dose of 1600 units is given as the one-time dose at Visit 3 (Week 0).

**Table 28. BIF Starting Weekly Dose Calculation - Participants with FBG ≤ 120 mg/dL. The maximum weekly dose should not exceed 1400 units. If the calculated weekly dose is greater than 1400 units, prescribe only 1400 units.**

| Step | Action | Example Participant B (FBG ≤120 mg/dL) |
|---|---|---|
| 1. | Review usual prior daily basal dose from lead-in period | 30 units/day |
| 2. | Calculate Starting Weekly Dose | 210 units/Week |
| | (Usual Daily Dose multiplied by 7 and round to nearest 10) | |

Visit 4 (Week 1): Starting Weekly Dose. If the participant experienced hypoglycemia in the previous week and met any of the criteria for a dose reduction listed in Table 29 subtract 40 units from the Starting Weekly Dose to obtain the dose for administration at Visit 4 (Week 1).

**Table 29. Hypoglycemia Dose Reduction for BIF.**

| Hypoglycemia Dose Reduction Criteria Based on BG Values | Unit Dose Decrease |
|---|---|
| ≥3 episodes ≤ 70 mg/dL (≤ 3.9 mmol/L) | 40 units |
| ≥1 nocturnal episode ≤ 70 mg/dL (3.9 mmol/L) | |
| ≥1 episode < 54 mg/dL (3.0 mmol/L) | |
| Any confirmed severe hypoglycemia | |

If the Hypoglycemia Dose Reduction criteria are not met, administer the Starting Weekly Dose.

The following steps illustrate an example calculation for Visit 4 (Week 1) of Starting Weekly Dose.

**Table 30. Example calculation of starting weekly dose.**

| Step | Action | Example Patient B |
|---|---|---|
| 1. | Review Starting Weekly Dose from Visit 3 (Week 0) calculations | 210 units/day |
| 2. | Determine if any of Hypoglycemia Dose Reduction criteria are met in prior week (Table 21): | a. If a Hypoglycemia Dose Reduction criterion is met, |
| | | ➢ Week 1 Dose = 210 - 40 = 170 units/week |
| | If yes, subtract 40 units from Starting Weekly Dose | |
| | | b. If no Hypoglycemia Dose Reduction criterion was met, the Week 1 Dose=210 units/week |
| | If not, administer Starting Weekly Dose | |

Visit 5 (Week 2): Titration to Week 77. If the participant experienced any documented hypoglycemia (BG ≤ 70 mg/dL) in the previous week, the dose should not be increased. If the participant experienced hypoglycemia in the previous week and met criterion for a dose reduction as described in Table 29, subtract 40 units from the previous weekly dose.

If no BG ≤70 mg/dL was reported, use the median FBG from the previous week to obtain the dose adjustment from the titration algorithm in Tables 31, 32 or 33 as appropriate given the participant's median FBG from the week prior to randomization and/or prestudy basal insulin dose. As indicated in Tables 31-33, if the median FBG is within the target range from 80 to 120 mg/dL inclusive, no change would be made to the dose from the previous week.

**Table 31. BIF Dose Adjustments for participants not described below in connection with Tables 32 or 33.**

| Median FBG | | BIF Dose adjustment |
|---|---|---|
| mg/dL | Mmol/L | Units |
| <80 | <4.4 | (-) 20 units (decrease) |
| 80-120 | 4.4-6.6 | No change to dose |
| 121-140 | 6.7-7.7 | (+) 20 units (increase) |
| >140 | >7.7 | (+) 40 units (increase) |

For study participants who enter the study with median FBG ≤120 mg/dL or prestudy basal insulin dose ≥ 10 units/day and <20 units/day, the investigator may adjust the BIF dose according to Table 32.

**Table 32. BIF Dose Adjustment with Median FBG ≤120 mg/dL or Prestudy Basal Insulin Dose ≥ 10 units/day and <20 units/day.**

| Median FBG | | BIF Dose adjustment |
|---|---|---|
| mg/dL | Mmol/L | Units |
| <80 | <4.4 | (-) 20 units (decrease) |
| 80-120 | 4.4-6.6 | No change to dose |
| 121-140 | 6.7-7.7 | (+) 10 units (increase) |
| >140 | >7.7 | (+) 20 units (increase) |

For study participants who enter the study with prestudy basal insulin dose <10 units/day, the investigator may adjust the BIF dose according to Table 33.

**Table 33. BIF Dose Adjustment with Prestudy Basal Insulin Dose <10 units/day.**

| Median FBG | | BIF Dose adjustment |
|---|---|---|
| mg/dL | Mmol/L | Units |
| <80 | <4.4 | (-) 20 units (decrease) |
| 80-120 | 4.4-6.6 | No change to dose |
| 121-140 | 6.7-7.7 | (+) 5 units (increase) |
| >140 | >7.7 | (+) 10 units (increase) |

In this section, dosing instructions for insulin degludec are provided for dose initiation at Visit 3 (Week 0), and administration of the daily dose at Visit 4 (Week 1) and subsequent dosing/dose adjustments throughout the treatment period.

Visit 3 - (Week 0): Randomization Visit Insulin Degludec Dose Determination from Equivalent of Prestudy Insulin Dose. Follow instructions in Table 34 to calculate the equivalent dose of insulin degludec to be administered by the participant once daily. Note: If a participant is on NPH twice-daily or U300 glargine, the prestudy basal insulin dose should be reduced by 20% when determining the once-daily dose of insulin degludec to initiate in the study.

**Table 34. Equivalent Dose of Insulin Degludec.**

| Step | Action | Example Participant C Prestudy insulin: glargine, U100 20 units once-daily | Example Participant D Prestudy insulin: NPH, 10 units twice-daily |
|---|---|---|---|
| 1. | Determine usual prior daily basal dose from lead-in period | 30 units/day | 10 units × 2 = 20 units/day |
| 2. | Determine daily dose of insulin degludec | 210 units/Week | 20 units - [20 × 0.2] units = 20 units - 4 units = 16 units/day |

Visit 4 (Week 1) to Visit 38 (Week 78). If the participant experienced any documented hypoglycemia (BG ≤70 mg/dL) in the previous week, the dose should not be increased. If the participant experienced hypoglycemia in the previous week that met dose reduction criterion, decrease the daily dose as described in Table 235.

**Table 35. Hypoglycemia Dose Reduction Criteria for Insulin Degludec**

| Hypoglycemia Dose Reduction Criteria Based on BG Values | Unit Dose Decrease |
|---|---|
| ≥3 episodes ≤ 70 mg/dL (≤ 3.9 mmol/L) | 2-4 units |
| ≥1 nocturnal episode ≤ 70 mg/dL (3.9 mmol/L) | |
| ≥1 episode < 54 mg/dL (3.0 mmol/L) | |
| Any confirmed severe hypoglycemia | |

If hypoglycemia dose reduction criteria are not met use the median FBG from the previous week to obtain the dose adjustment from the titration algorithm in Tables 36 or 37. For participants entering the study with prestudy basal insulin doses ≥ 10 units/day, the investigator may adjust the degludec dose according to Table 36 below.

**Table 36. Insulin degludec dose adjustment for participants with prestudy basal insulin dose ≥ 10 units/day.**

| Median FBG | | BIF Dose adjustment |
|---|---|---|
| mg/dL | Mmol/L | Units |
| <80 | <4.4 | (-) 2 units (decrease) |
| 80-120 | 4.4-6.7 | No change to dose |
| 121-140 | 6.7-7.8 | (+) 4 units (increase) |
| 141-180 | 7.8-10.0 | (+) 6 units (increase) |
| >180 | >10 | (+) 8 units (increase) |

For participants entering the study with prestudy basal insulin doses < 10 units/day, the investigator may adjust the degludec dose according to Table 37 below.

**Table 37. Insulin degludec dose adjustment for participants with prestudy basal insulin dose < 10 units/day.**

| Median FBG | | BIF Dose adjustment |
|---|---|---|
| mg/dL | Mmol/L | Units |
| <80 | <4.4 | (-) 2 units (decrease) |
| 80-120 | 4.4-6.7 | No change to dose |
| 121-140 | 6.7-7.8 | (+) 1 units (increase) |
| 141-180 | 7.8-10.0 | (+) 2 units (increase) |
| >180 | >10 | (+) 3 units (increase) |

Objectives and endpoints of the study are set forth in Table 38 below.

**Table 38. Objectives and Endpoints. Abbreviations: DTSQ = Diabetes Treatment Satisfaction Questionnaire - change version; CGM = continuous glucose monitoring; HbA1c = Hemoglobin A1c; SMBG = self-monitoring of blood glucose; SIM-Q = Simplicity Questionnaire; T2D = type 2 diabetes; TRIM-D = Treatment-Related Impact Measure - Diabetes.**

| Objectives | Endpoints |
|---|---|
| Primary | |
| To demonstrate noninferiority to insulin degludec on glycemic control | Change from baseline in HbA1c at Week 26 |

| Secondary | |
|---|---|
| To demonstrate superiority to insulin degludec in the selected parameters of glycemic control | • Change from baseline in HbA1c at Week 26 |
| | • The event rate of participant-reported clinically significant nocturnal hypoglycemia (<54 mg/dL or severe) during treatment phase up to Week 78 |
| | • Time in glucose range between 70 and 180 mg/dL inclusive measured by CGM collected session prior to Week 26 |
| To investigate efficacy compared with insulin degludec in additional parameters of glycemic control | • Change from baseline in HbA1c at Weeks 52 and 78 |
| | • Change from baseline in fasting glucose measured by SMBG at Weeks 26, 52, and 78 |
| | • Glucose variability measured during the CGM session prior to Weeks 26, 52, and 78 |
| | • Time in glucose range between 70 and 180 mg/dL inclusive measured during the CGM session prior to Weeks 52 and 78 |
| | • Insulin dose at Weeks 26, 52, and 78 |
| To investigate safety compared with insulin degludec | • Incidence and rate of composite of Level 2 and 3 hypoglycemia events during treatment period |
| | • Body weight change from baseline to Weeks 26, 52, and 78 |
| | • Time in hypoglycemia with glucose <54 mg/dL during the CGM session prior to Weeks 26, 52, and 78 |
| | • Time in hyperglycemia range defined as glucose >180 mg/dL during the CGM session prior to Weeks 26, 52, and 78 |
| To evaluate treatment impact between BIF and insulin degludec based on patient-reported outcomes questionnaires | • Change from baseline in TRIM-D at Weeks 26, 52, and 78 |
| | • DTSQ change from baseline at Weeks 26, 52, and 78 |
| To investigate the treatment impact of BIF compared with insulin degludec on other measures of efficacy, safety, and patient-reported outcomes | Efficacy |
| | • Percentage of participants achieving HbA1c < 7% at Weeks 26, 52, and 78 |
| | • Percentage of participants achieving HbA1c < 7% at Week 26 without nocturnal hypoglycemia during treatment phase up to Week 26 |
| | • Percentage of participants achieving HbA1c ≤ 6.5% at Weeks 26, 52, and 78 |
| | • Change from baseline in fasting serum glucose at Weeks 26, 52, and 78 |
| | Safety |
| | • Incidence and rate of Level 2 hypoglycemia events during treatment period |
| | • Incidence and rate of Level 3 hypoglycemia events during treatment period |
| | • Incidence of positive treatment-emergent antibody of BIF |
| | Patient-Reported Outcomes |
| | • Change from baseline in SIM-Q at Week 26 |
| | • Frequency of responses to "Basal Insulin Experience: Likelihood of incorporating into routine" at Weeks 26 and 78 |
| | • Frequency of responses to "Basal Insulin Experience: Preference" at Weeks 26 and 78 |

### Insulin-naive T2DM patients

A Phase 3, parallel-design, open-label, randomized control trial is designed to evaluate the efficacy and safety of BIF as a weekly basal insulin compared to degludec, in insulin naive adults with T2D, inadequately controlled with oral anti-hyperglycemic medications (OAMs), with or without GLP-1RA. Participants will continue prior stable therapy with 0 up to 3 allowed noninsulin diabetes medications during the study.

Prefilled insulin pens that display and deliver the insulin dose in total weekly units, will be provided for the subcutaneous administration of BIF once weekly, or in daily units for the administration of insulin degludec once daily, based on the participant's randomly assigned treatment. In both the treatment arms, participants will be provided with glucometers for self-monitoring of blood glucoses, instructed about hypoglycemia recognition and treatment, and trained on protocol-related tasks. Investigators will determine participant's insulin doses according to protocol and oversee the dose adjustments to achieve blood glucose target while avoiding hypoglycemia. Primary endpoint for the study is at 52 weeks. Treatment duration is 52 weeks, and the initiation of rescue therapies will start after 16 weeks.

Regular periodic review of blinded safety data will be conducted by the study team (following the study's Trial level Safety Review Plan), and an external Data Monitoring Committee will review unblinded safety data as it evaluates safety across all BIF Phase 3 studies.

Key design features include those set forth in Table 39 below.

**Table 39. Abbreviations: BG = blood glucose; CGM = continuous glucose monitoring; FBG = fasting blood glucose; FDA = Food and Drug administration; FGM = flash glucose monitoring; GLP-1 RA = glucagon-like peptide-1 receptor agonist; HbA1c = glycated hemoglobin A1c; LY = BIF; MIDD = Model-Informed Drug Development; NI = noninferiority; OAM = oral antihyperglycemic medication; PD = pharmacodynamics; PK = pharmacokinetics; PwT2D = people with type 2 diabetes mellitus; SD = standard deviation.**

| | **Design and features** | **Rationale** |
|---|---|---|
| **Study duration** | 60 weeks | This duration provides sufficient time for glucose levels to stabilize following initiating and optimizing titration of the study insulin therapy and would also allow evaluation of efficacy and safety with long-term use. |
| **Comparator** | Degludec | It is a well-characterized basal insulin administered as a once-daily injection. It has the longest half-life among other marketed basal insulins. |
| **Randomization** | 1:1 (BIF once weekly: degludec once daily) | |
| **Patient Population** | • PwT2D ≥18 years of age at screening | Criteria will allow assessment of BIF (compared with degludec) in insulin naive adult PwT2D on non-insulin anti-hyperglycemic medications with glycemic control that ranges from fairly good to poor at baseline. |
| | • Patients on a maximum of 3 anti-hyperglycemic medications (OAMs+/-GLP-1 RA) | |
| | • Patients need to be on stable doses at least 3 months prior to screening and be willing to continue stable dosing throughout the study. | |
| | • HbA1c 7.0%-10.5% inclusive | |
| | • Body mass index (BMI) ≤45 kg/m2, inclusive, with no significant weight gain or loss in the past 3months (≥5%). | |
| | • A more detailed list of the Inclusion and Exclusion criteria is provided below. | |
| **Exposure** | N=888 | Approximately 888 participants will be randomized to BIF and degludec in 1:1 ratio. With the assumption of 15% drop off at Week 52, approximately 377 and 377 participants will complete 52 weeks of treatment on LY and degludec, respectively. The primary objective is to show a NI based on a 0.4% margin. However, the study is powered for a 0.3% NI margin to meet global regulatory requirements. Using a 2-sided 0.05 level test and assuming 1.1% SD, 0.3% NI margin, and no treatment difference for HbA1c change from baseline, this sample size will provide at least 95% statistical power to show noninferiority of BIF to degludec. |
| | LY=444, degludec=444 | |
| **Method of delivery** | KwikPen Insulin Delivery device | The BIF KwikPen(s) will be similar to the BASAGLAR KwikPen that has been adapted to deliver a weekly dose of BIF in 5-unit increments. |
| **Dosing regimen** | Instructions for initiating and titrating BIF are described below, and were informed by findings from Phase 1 and 2 studies and PK/PD modeling. Insulin doses and recommended dose adjustments in the dosing algorithms will be aligned with the pen device labeling in 5-unit increments. | Appropriate starting dose and dose adjustment guidance for treat-to-target approach with titratable insulins are used in clinical practice. Emerging data with BIF and PK/PD modeling will guide dose titrations. |
| | Instructions for initiating and titrating the treatment comparator will follow the prescribing information and standards of care for once-daily insulin degludec. | |

Criteria for inclusion in the study include the following: 1. Are at least 18 years of age at screening (or older per local regulations); 2. Have a diagnosis of type 2 diabetes mellitus (T2D) according to the WHO criteria; 3. Have a baseline glycated hemoglobin A1c (HbA1c) value of 7.0% to 10.5%, inclusive, at screening; 4. Acceptable noninsulin diabetes therapies may include 0 to up to 3 of the following: sulfonylureas, thiazolidinediones (TZDs), dipeptidyl peptidase (DPP-4) IV inhibitors, sodium-glucose co-transporter (SGLT)-2 inhibitors, biguanides (e.g., metformin), alpha-glucosidase inhibitors, or glucagon-like peptide-1(GLP-1) receptor agonists; 5. Insulin naive. However, short term insulin treatment for a maximum of 14 days prior to the day of screening is allowed, as is prior insulin treatment for gestational diabetes; 6. Have a BMI ≤45 kg/m2 at screening with no significant weight gain or loss in the past 3 months (≥5%); 7. In the investigator's opinion, are well-motivated, capable, and willing to learn how to self-inject treatment, as required for this protocol.

Individuals are excluded from the study if any of the following criteria apply: 1. Have a diagnosis of type 1 diabetes mellitus, latent autoimmune diabetes, or specific type of diabetes other than T2D (for example, monogenic diabetes, diseases of the exocrine pancreas, drug-induced or chemical-induced diabetes); 2. Have received any of the following nonallowed diabetes medication within prior 30 days including glinides, pramlintide, basal insulin, prandial insulin, insulin mixtures, inhaled insulins, continuous subcutaneous insulin infusion therapy; 3. Have a history of greater than 1 episode of ketoacidosis or hyperosmolar state/coma requiring hospitalization in the 6 months prior to screening; 4. Have had any episodes of severe hypoglycemia in the 6 months prior to screening; 5. Have hypoglycemia unawareness in the opinion of the investigator; 6. Cardiovascular (CV): have had New York Heart Association Class IV heart failure or any of the following CV conditions in the past 3 months prior to screening: acute myocardial infarction, cerebrovascular accident (stroke), or coronary bypass surgery; 7. Gastrointestinal: have undergone gastric bypass (bariatric) surgery, restrictive bariatric surgery (for example, Lap-Band^{®}), or sleeve gastrectomy within 1 year prior to screening or have presence of clinically significant gastroparesis in the opinion of the investigator; 8. Hepatic: have acute or chronic hepatitis, cirrhosis, or obvious clinical signs or symptoms of any other liver diseases, except nonalcoholic fatty liver disease (NAFLD) (i.e., study participants with NAFLD are eligible for participation), and/or have elevated liver enzyme measurements, as determined by the central laboratory at screening: Total bilirubin >2x the upper limit of normal (ULN), Alanine aminotransferase (ALT)/ serum glutamic pyruvic transaminase (SGPT)>2.5x ULN, or Aspartate aminotransferase (AST)/ serum glutamic oxaloacetic transaminase (SGOT) > 2.5x ULN, Alkaline phosphatase (ALP) > 2.5x ULN; 9. Renal: have a history of renal transplantation, are currently receiving renal dialysis, or have an estimated glomerular filtration rate(eGFR) <20 mL/min/1.73 m2, calculated by the Chronic Kidney Disease-Epidemiology equation, as determined by the central laboratory at screening; 10. Have active or untreated malignancy, have been in remission from clinically significant malignancy (other than basal cell or squamous cell skin cancer) for less than 5 years, or are at increased risk for developing cancer or a recurrence of cancer in the opinion of the investigator; 11. Have known hypersensitivity or allergy to any of the study medications or their excipients; 12. Have any other serious disease or condition (for example, known drug or alcohol abuse or psychiatric disorder) that, in the opinion of the investigator, would pose a significant risk to the study participant, preclude the study participant from following and completing the protocol; 13. Have evidence of current or recent within 6 months timeframe history of any substance use disorder(s) of any severity as defined by the DSM-5 in the opinion of the investigator, except disorders of nicotine or caffeine use; 14. Hematologic: have had a blood transfusion or severe blood loss within 90 days prior to Visit 1, or have known hemoglobinopathy, hemolytic anemia, sickle cell anemia, or any other traits of hemoglobin abnormalities known to interfere with the measurement ofHbA1c in the opinion of the investigator; 15. Are receiving chronic (>14 days) systemic glucocorticoid therapy (excluding topical, intraocular, intranasal, or inhaled preparations) or have received such therapy for>14 days within the month preceding screening.

Timing and instructions for administration of BIF and insulin degludec will be similar to that described above for the Phase 3 study on T2DM patients being treated with a daily basal insulin except that participants in the present study do not have a pre-study basal insulin dose, so a standardized expected weekly maintenance dose must be used that is expected to be appropriate and tolerated by this patient population.

For participants randomized to BIF, the expected weekly maintenance dose will be 100 U, and a 3X loading dose will be used, so the initial dose at Visit 4 (Week 1) will be 300 U. The need for any adjustments for weekly maintenance doses thereafter will be determined according to the criteria described above for the Phase 3 study on T2DM patients being treated with a daily basal insulin, subject to the hypoglycemia criteria also described above.

For participants randomized to insulin degludec, the initial dose administered on day 1, will be 10 U, and adjustments will be made on a weekly basis thereafter according to the criteria described above for the Phase 3 study on T2DM patients being treated with a daily basal insulin.

Efficacy and safety objectives, assessments and endpoints are set forth in Table 40 below.

**Table 40. Efficacy and safety objectives, assessments and endpoints**

| | | | |
|---|---|---|---|
| Objectives | Primary: | | The primary efficacy measurement is HbA1c, a widely used measure of glycemic control that reflects a cumulative history of glucose levels in the preceding 2 to 3 months. It has been found to correlate well with the risk of long-term diabetes complications. It is a well-accepted measure in assessing a drug's glucose-lowering efficacy. The other secondary objectives provide complementary information about glycemic control. Hypoglycemia, adverse events, and immunogenicity will be assessed to characterize safety. |
| | To demonstrate noninferiority to degludec for the change in HbA1c from baseline to 52 weeks, with a noninferiority margin of 0.4%. | | |
| | Key Secondary: | | |
| | 1.To demonstrate noninferiority to insulin degludec in HbA1c change from baseline to Wk 52 (0.4% NI) for the subpopulation of participants with concomitant oral antihyperglycemic medications only without GLP-1 | | |
| | 2.To demonstrate noninferiority to insulin degludec in HbA1cchange from baseline to Wk 52 (0.4% NI) for the subpopulation of participants with concomitant oral antihyperglycemic medications with GLP-1 RA. | | |
| | 3.Change from baseline inHbA1c for superiority at Week 52 | | |
| | 4.Time in range (TIR) between 70 and 180 mg/dL inclusive measured by CGM at Week 52 | | |
| | 5.The event rate of participant-reported clinically significant nocturnal hypoglycemia (<54mg/dL or severe) from baseline to Week 52. | | |
| Key safety assessments | | • Adverse events | |
| | | • Hypoglycemia | |
| | | • Hypersensitivity/ Injection site reactions | |
| | | • Immunogenicity | |

### T2DM patients being treated with MDI

A Phase 3, parallel-design, open-label, randomized control trial is designed to evaluate the efficacy and safety of BIF compared to glargine in patients with T2D who are on once- or twice-daily basal insulin and at least two injections per day of prandial insulin prior to entering the study. Participants will continue prior stable therapy with 0 to 3 allowed noninsulin diabetes medications during the study.

Prefilled insulin pens that display and deliver the insulin dose in total weekly units will be provided for the subcutaneous administration of BIF once-weekly or in daily units for administration of insulin glargine once daily, based on the participant's randomly assigned treatment. In both the treatment arms, participants will be provided with glucometers for self-monitoring of blood glucoses, instructed about hypoglycemia recognition and treatment, and trained on protocol-related tasks. Investigators will determine participant's insulin doses as described below and oversee the dose adjustments to achieve blood glucose target while avoiding hypoglycemia.

Key design features include those set forth in Table 41 below.

**Table 41. Abbreviations: BG = blood glucose; CGM = continuous glucose monitoring; FBG = fasting blood glucose; FDA = Food and Drug administration; FGM = flash glucose monitoring; GLP-1 RA = glucagon-like peptide-1 receptor agonist; HbA1c = glycated hemoglobin A1c; MIDD = Model-Informed Drug Development; NI = noninferiority; OAM = oral antihyperglycemic medication; PD = pharmacodynamics; PK = pharmacokinetics; PwT2D = people with type 2 diabetes mellitus; SD = standard deviation.**

| | **Design and features** | **Rationale** |
|---|---|---|
| **Study duration** | 26 weeks | This duration provides sufficient time for glucose levels to stabilize following the switch from prestudy insulin therapy to the study insulins. |
| **Comparator** | glargine | It is a well-characterized basal insulin administered as a once-daily injection. |
| **Randomization** | 1:1 (BIF once weekly: glargine once daily) | |
| **Patient Population** | • PwT2D ≥18 years of age at screening | Criteria will allow assessment of BIF (compared with glargine) in adult PwT2D on basal insulin and prandial insulin with glycemic control that ranges from fairly good to poor at baseline. |
| | • Patients on a maximum of 3 anti-hyperglycemic medications (OAMs+/-GLP-1 RA) | |
| | • Patients on basal insulin with at least 2 daily injections of prandial insulin, with or without OAM or GLP-1 RA | |
| | • HbA1c 7.0%-10% inclusive | |
| | • Once- or twice-daily basal insulin | |
| | • A more detailed list of the Inclusion and Exclusion criteria is provided below. | |
| **Exposure** | N=670 | Approximately 670 participants will be randomized to BIF and glargine in 1:1 ratio. With the assumption of 15% drop-off at Week 26, approximately 285 participants will complete 26 weeks of treatment on BIF or glargine. The primary objective is to show a NI based on a 0.4% or 0.3% margin depending on the regulatory requirements. The study is powered for a 0.3% NI margin to meet global regulatory requirements. Using a 2-sided 0.05 level test and assuming 1.1% SD, 0.3% NI margin, and no treatment difference for HbA1c change from baseline, this sample size will provide at least 90% statistical power to show noninferiority of BIF to glargine. |
| | BIF=335, glargine=335 | |
| **Method of** | KwikPen Insulin | The BIF KwikPen(s) will be similar to the BASAGLAR KwikPen that has been adapted to deliver a weekly dose of BIF in 5-unit increments. |
| **delivery** | Delivery device | |
| **Dosing regimen** | Instructions for initiating and titrating BIF are described below, and were informed by findings from Phase 1 and 2 studies and PK/PD modeling. | Appropriate starting dose and dose adjustment guidance for treat-to-target approach with titratable insulins are used in clinical practice. Emerging data with BIF and PK/PD modeling will guide dose titrations. |
| | The starting doses, dose titrations, frequency of titration, and BG targets for BIF treatment are described below. Insulin doses and recommended dose adjustments in the dosing algorithms will be aligned with the pen device labeling in 10-unit increments. | |
| | Instructions for initiating and titrating the treatment comparator will follow the prescribing information and standards of care for once-daily insulin glargine. | |

Inclusion criteria include the following: 1. Are at least 18 years of age at screening (or older per local regulations); 2. Have a diagnosis of type 2 diabetes mellitus (T2D) according to the WHO criteria currently treated with basal insulin and at least two injections of prandial insulin per day; 4. Less than or equal to 2 units/kg/day of total daily insulin at screening; 5. Have a baseline glycated hemoglobin A1c (HbA1c) value of 7.0% to 10%, inclusive, at screening; 6. Have been treated with a stable regimen of one of the following basal insulins used according to local product label with or without noninsulin diabetes therapy for at least 90 days prior to screening: once daily U100 or U200 insulin degludec; once daily U100 or U300 insulin glargine; once or twice daily U100 insulin detemir; once or twice daily human insulin Neutral Protamine Hagedorn; 7. Have been treated with a stable regimen of at least twice daily dosing of one of the following insulins used according to local product label for at least 90 days prior to screening (one dose of prandial insulin must occur prior to the evening meal): insulin lispro (U100 and U200); insulin lispro-aabc (U100 or U200); insulin aspart (U100; including Fiasp and Novolog); insulin glulisine (U100); regular insulin (U100); acceptable noninsulin diabetes therapies may include 0 to up to 3 of the following: dipeptidyl peptidase IV inhibitors, sodium-glucose co-transporter-2 inhibitors, biguanides (e.g., metformin), or glucagon-like peptide-1 receptor agonists. Note: All noninsulin diabetes therapies must be used in accordance with the corresponding local product label at the time of screening, and participants should be willing to continue stable dosing throughout the study according to the protocol. 8. Have a BMI ≤45 kg/m2, inclusive with no significant weight gain or loss in the past 3 months (≥5%).

Exclusion criteria include the following: 1. Have had a significant weight gain or loss in the past 3 months in the investigator's opinion (for example, ≥5%); 2. Have a diagnosis of type 1 diabetes mellitus or latent autoimmune diabetes, or specific type of diabetes other than T2D (e.g., monogenic diabetes, diseases of the exocrine pancreas, drug induced or chemical-induced diabetes); 3. Are currently receiving any of the following insulin therapies (outside of pregnancy) anytime in the past 90 days except for short-term treatment of acute conditions, and up to a maximum of 4 continuous weeks: insulin mixtures, Affreza (inhaled regular human insulin), continuous subcutaneous insulin infusion therapy, Regular insulin U500; 4. Have received any of the following nonallowed diabetes medication within prior 90 days including glinides, sulfonylureas, pramlintide, alpha-glucosidase inhibitors or thiazolidinediones; 5. Have a history of greater than 1 episode of ketoacidosis or hyperosmolar state/coma requiring hospitalization in the 6 months prior to screening; 6. Have had any episodes of severe hypoglycemia within the 6 months prior to screening; 7. Have hypoglycemia unawareness in the opinion of the investigator; 8. No intention of cgm use changes (e.g., initiation, stopping, changing device) during the study; 9. Cardiovascular (CV): have had New York Heart Association Class IV heart failure or any of the following CV conditions in the past 3 months prior to screening: acute myocardial infarction, cerebrovascular accident (stroke), or coronary bypass surgery; 10. Gastrointestinal: have undergone gastric bypass (bariatric) surgery, restrictive bariatric surgery (e.g., Lap-Band^{®}), or sleeve gastrectomy within 1 year prior to screening or have presence of clinically significant gastroparesis in the opinion of the investigator; 11. Hepatic: have acute or chronic hepatitis, or obvious clinical signs or symptoms of any other liver disease except nonalcoholic fatty liver disease (NAFLD) (i.e., study participants with NAFLD are eligible for participation), and/or have elevated liver enzyme measurements, as determined by the central laboratory at screening and as follows: Total bilirubin >2x the upper limit of normal (ULN), with the exception of previous diagnosis of Gilbert's Disease; Alanine aminotransferase/serum glutamic pyruvic transaminase >2.5x ULN; or Aspartate aminotransferase/serum glutamic oxaloacetic transaminase > 2.5x ULN; 12. Renal: have a history of renal transplantation, are currently receiving renal dialysis, or have an estimated glomerular filtration rate <30 mL/min/1.73 m2, calculated by the Chronic Kidney Disease-Epidemiology equation, as determined by the central laboratory at screening; 13. Have active or untreated malignancy, have been in remission from clinically significant malignancy (other than basal cell or squamous cell skin cancer) for less than 5 years, or are at increased risk for developing cancer or a recurrence of cancer in the opinion of the investigator; 13. Hematologic: have had a blood transfusion or severe blood loss within 90 days prior to Visit 1 or have known hemoglobinopathy, hemolytic anemia or sickle cell anemia, or any other traits of hemoglobin abnormalities known to interfere with the measurement of HbA1c in the opinion of the investigator; 14. Are receiving chronic (>14 days) systemic glucocorticoid therapy (excluding topical, intraocular, intranasal, or inhaled preparations) or have received such therapy for >14 days within the month preceding screening.

Instructions for initiating and titrating treatment with BIF are provided as described in Tables 42 and 43 below:

**Table 42. Instructions for initiating treatment with BIF.**

| Baseline median FPG | Initial Dose |
|---|---|
| ≧ 120 mg/dL | prior daily basal dose (U) x 7 x 3 |
| ≤ 120 mg/dL | prior daily basal dose (U) x 7 (no loading dose multiplier) |

**Table 43. Instructions for determination of BIF weekly maintenance doses. ^{a} Reductions may be in the range of 10-20 units at the discretion of the investigator depending on the participant's FBG value and other individual clinical characteristics.**

| Median FBG (mg/dL) | BIF adjustment (units) | |
|---|---|---|
| | Baseline | Baseline |
| | FG ≤ 120 mg/dL or daily basal dose < 20 units | FG > 120 and daily basal dose ≥ 20 units |
| <80 | -10 to 20^{a} | -20 |
| 80-120 | No change | No change |
| 121-140 | +10 | +20 |
| > 140 | +20 | +40 |

Efficacy and safety objectives, assessments and endpoints are set forth in Table 44 below.

**Table 44. Efficacy and safety objectives, assessments and endpoints**

| | Design and Features | | Rationale |
|---|---|---|---|
| Objectives | Primary: | | The primary efficacy measurement is HbA1c, a widely-used measure of glycemic control that reflects a cumulative history of glucose levels in the preceding 2 to 3 months. It has been found to correlate well with the risk of long-term diabetes complications. It is a well-accepted measure in assessing a drug's glucose-lowering efficacy. The other secondary objectives provide complementary information about glycemic control. Hypoglycemia, adverse events, and immunogenicity will be assessed to characterize safety. |
| | To demonstrate noninferiority to glargine for the change in HbA1c from baseline to 26 weeks. | | |
| | Key Secondary: | | |
| | | • Change in HbA1c from baseline | |
| | | • <7% without nocturnal hypoglycemia (<54 mg/dl or severe) | |
| | • The event rate of participant-reported clinically significant nocturnal hypoglycemia (<54 mg/dl or severe) during treatment phase up to Week 26 | | |
| Key safety assessments | | • Adverse events | |
| | | • Hypoglycemia | |
| | | • Hypersensitivity/ Injection site reactions | |
| | | • Immunogenicity | |

### T1D patients

A Phase 3, parallel-design, open-label, randomized control trial is designed that will evaluate the efficacy and safety of BIF compared to degludec in participants with T1D who are treated with basal-bolus MDI therapy prior to entering the study. A study description with more details is provided in Table 45 below. The choice of study population and insulin comparator is supported by available efficacy and safety data from the Phase 2 study described above in a similar population that compared BIF to degludec. Prefilled insulin pens that display and deliver the insulin dose in total weekly units will be provided for the subcutaneous administration of BIF once-weekly or in daily units for administration of insulin degludec once daily, based on the participant's randomly assigned treatment. In both treatment arms, participants will be provided with unblinded CGM and glucometers for diabetes management, instructed about hypoglycemia recognition and treatment, and trained on protocol-related tasks. Investigators will determine participant's insulin doses according to protocol and oversee the dose adjustments to achieve glucose targets while avoiding hypoglycemia.

Key design features include those set forth in Table 45 below.

**Table 45. Abbreviations: CGM = continuous glucose monitoring; FDA = Food and Drug administration; HbA1c = glycated hemoglobin A1c; MIDD = Model-Informed Drug Development; NI = noninferiority; PD = pharmacodynamics; PK = pharmacokinetics; SD = standard deviation.**

| | **Design and features** | **Rationale** |
|---|---|---|
| **Study duration** | 52 weeks | This duration provides sufficient time for glucose levels to stabilize following the switch from prestudy insulin therapy to the study insulins. |
| **Comparator** | degludec | It is a well-characterized basal insulin administered as a once-daily injection. It has the longest half-life among other marketed basal insulins. |
| **Randomization** | 1:1 (BIF once weekly: degludec once daily) | |
| **Patient population** | • ≥18 years of age | Criteria will allow assessment of BIF (compared with degludec) in adults with T1D on basal-bolus MDI therapy. |
| | • T1D for at least 1 year | |
| | • Treated with basal-bolus insulin MDI therapy | |
| | • HbA1c 7.0%-10% inclusive | |
| | • A more detailed list of the Inclusion and Exclusion criteria is provided below. | |
| **Patient population** | N=670 | Approximately 670 participants will be randomized to BIF and degludec in 1:1 ratio. With the assumption of 15% dropout at Week 26, approximately 285 and 285 participants will complete 26 weeks of treatment on BIF and degludec, respectively. The primary objective is to show a NI based on a 0.4% margin. |
| | BIF=335, degludec=335 | |
| | | However, the study is powered for a 0.3% NI margin to meet global regulatory requirements. Using a 2-sided 0.05 level test and assuming 1.1% SD, 0.3% NI margin, and no treatment difference for HbA1c change from baseline, this sample size will provide approximately 90% power to show noninferiority of BIF to degludec. |
| **Method of delivery** | KwikPen Insulin | The BIF KwikPen(s) will be similar to the BASAGLAR KwikPen that has been adapted to deliver a weekly dose of BIF in 5-unit increments. |
| | Delivery device | |
| **Dosing regimen** | Instructions for initiating and titrating BIF (including treat-to-target dosing algorithms) are described below, and were be informed by findings from prior clinical study and PK/PD modeling. Insulin doses and recommended dose adjustments in the dosing algorithms will be aligned with the pen device labeling in 5-unit increments. | Appropriate starting dose and dose adjustment guidance for treat-to-target approach with titratable insulins are used in clinical practice. Emerging data with this novel once-weekly insulin and PK/PD modeling will guide dose titrations. |
| | Instructions for initiating and titrating the treatment comparator will follow the prescribing information and standards of care for once-daily insulin degludec. | |

Inclusion criteria include the following: 1. Are at least 18 years of age at screening (or older per local regulations); 2. Have a diagnosis of type 1 diabetes mellitus (T1D) according to the WHO criteria for at least 1 year prior to screening; 3. Have hemoglobin A1c (HbA1c) value of 7.0% to 10%, inclusive, at screening; 4. Have been treated with basal-bolus insulin analog MDI therapy according to the local product label for at least 90 days prior to screening, including: basal insulin analog (glargine U-100, glargine U-300, degludec, detemir) in combination with bolus insulin analog (insulin lispro, insulin aspart, insulin glulisine, Fiasp, Lyumjev) with meals; and 5. Have a BMI ≤35.0 kg/m2.

Exclusion criteria include the following: 1. Have a diagnosis of type 2 diabetes mellitus or latent autoimmune diabetes; 2. Have a history of more than 1 episode of diabetic ketoacidosis or hyperosmolar state/coma requiring hospitalization within the 6 months prior to screening; 3. Have a history of more than 1 episode of severe hypoglycemia (defined as requiring assistance due to neurologically disabling hypoglycemia) within the 6 months prior to screening; 4. Have hypoglycemia unawareness in the opinion of the investigator; 5. Have excessive insulin resistance defined as having received a total daily dose of insulin >1.5 units/kg at the time of screening; 6. Cardiovascular (CV): have had New York Heart Association Class IV heart failure or any of the following CV conditions in the past 3 months prior to screening: acute myocardial infarction, cerebrovascular accident (stroke), or coronary bypass surgery; 7. Gastrointestinal: have undergone gastric bypass (bariatric) surgery, restrictive bariatric surgery (e.g., Lap-Band^{®}), or sleeve gastrectomy within 1 year prior to screening; Have presence of clinically significant gastroparesis in the opinion of the investigator; 8. Hepatic: have acute or chronic hepatitis, cirrhosis, or obvious clinical signs or symptoms of any other liver disease, except nonalcoholic fatty liver disease (NAFLD) (i.e., study participants with NAFLD are eligible for participation), and/or have elevated liver enzyme measurements, as determined by the central laboratory at screening and as follows: Total bilirubin >2x the upper limit of normal (ULN); Alanine aminotransferase/serum glutamic pyruvic transaminase >2.5x ULN, or Aspartate aminotransferase/serum glutamic oxaloacetic transaminase > 2.5x ULN, Alkaline phosphatase (ALP) > 2.5x ULN; 9. Renal: have a history of renal transplantation, are currently receiving renal dialysis, or have an estimated glomerular filtration rate <30 mL/min/1.73 m2, calculated by the Chronic Kidney Disease-Epidemiology equation, as determined by the central laboratory at screening; 10. Have active or untreated malignancy, have been in remission from clinically significant malignancy (other than basal cell or squamous cell skin cancer) for less than 5 years, or are at increased risk for developing cancer or a recurrence of cancer in the opinion of the investigator; 11. Hematologic: have had a blood transfusion or severe blood loss within 3 months prior to Visit 1 or have known hemoglobinopathy, hemolytic anemia or sickle cell anemia, or any other traits of hemoglobin abnormalities known to interfere with the measurement of HbA1c in the opinion of the investigator; 12. Have been on an insulin treatment regimen that includes NPH insulin, U-500 insulin, regular human insulin, or any premixed insulins within 90 days prior to screening (Visit 1); 13. Have used insulin human inhalation powder (Afrezza) within 90 days prior to screening (Visit 1); 14. Have used continuous subcutaneous insulin infusion (CSII) therapy within 90 days prior to screening (Visit 1); 15. Receiving any oral or injectable medication intended for the treatment of diabetes mellitus other than insulins in the 90 days prior to screening (Visit 1); 16. Are receiving chronic (>14 days) systemic glucocorticoid therapy (excluding replacement therapy for adrenal insufficiency; topical, intraocular, intranasal, or inhaled preparations) or have received such therapy for >14 days within the month preceding screening.

Instructions for initiating and titrating BIF (including treat-to-target dosing algorithms) are described below in Tables 46 and 47:

**Table 46. Instructions for determination of loading dose and first weekly maintenance dose.**

| Baseline FBG | Loading Dose | First Weekly maintenance dose |
|---|---|---|
| ≤ 140 mg/dL | Prior daily dose (U) x 7 x3 | Prior daily dose (U) x 7 |
| 141 - 160 mg/dL | [Prior daily dose x 1.1 to 1.2 (i.e., increase dose by 10 to 20%)] x 7 x3 | [Prior daily dose x 1.1 to 1.2 (i.e., increase dose by 10 to 20%)] x 7 |
| > 160 mg/dL | [Prior daily dose x 1.2 to 1.3 (i.e. increase dose by 20 to 30%)] x 7 x3 | [Prior daily dose x 1.2 to 1.3 (i.e., increase dose by 20 to 30%)] x 7 |

**Table 47. Dose adjustments for subsequent weekly maintenance doses.**

| Median FPG (mg/dL) | Adjustment | |
|---|---|---|
| | Current dose < 100 U | Current dose > 100 U |
| < 80 | Previous lower dose | Previous lower dose |
| 80-120 | No change | No change |
| 121-150 | + 5 U | + 10 U |
| 151-180 | + 10 U | + 20 U |
| >180 | +20U | +30U |

In addition, guidelines are provided for reducing the dose to the previous lower dose for certain hypoglycemia events.

Insulin degludec is titrated to FG target of 80-120 mg/dL using a modified Riddle treat-to-target algorithm.

Efficacy and safety objectives, assessments and endpoints are set forth in Table 48 below.

**Table 48. Efficacy and safety objectives, assessments and endpoints.**

| | Design and Features | | Rationale |
|---|---|---|---|
| Objectives | Primary: | | The primary efficacy measurement is HbA1c, a widely-used measure of glycemic control that reflects a cumulative history of glucose levels in the preceding 2 to 3 months. It has been found to correlate well with the risk of long-term diabetes complications. It is a well-accepted measure in assessing a drug's glucose-lowering efficacy. The other secondary objectives provide complementary information about glycemic control. Hypoglycemia, adverse events, and immunogenicity will be assessed to characterize safety. |
| | To demonstrate noninferiority to degludec for the change in HbA1c from baseline. | | |
| | Key Secondary: | | |
| | | • Change from baseline in HbA1c | |
| | | • Event rate of participant-reported clinically significant nocturnal hypoglycemia (<54 mg/dL or severe) during treatment phase up to Week 52 | |
| | | • Time in normal glycemia range between 70 and 180 mg/dL inclusive measured by CGM collected during the CGM session | |
| Key safety assessments | | • Hypoglycemia | |
| | | • Adverse events | |
| | | • Allergic/hypersensitivity reactions | |
| | | • Immunogenicity | |
| | | • Injection site reactions | |

### Other studies for T2DM patients

In other studies, key inclusion criteria include a prior diagnosis of diabetes on stable background therapy, at least 18 years of age, a baseline HbA1c value of approximately 7 to 10%, and a body mass index (BMI) between 20 and 45 kg/m2. Inclusion criteria and background therapy will vary according to the study population.

The primary objective of these studies is to investigate the effects of BIF on glycemic control compared with a marketed basal insulin like insulin glargine or insulin degludec. The associated primary end point is to demonstrate noninferiority of BIF compared with one of these basal insulins for the change in HbA1c from baseline. Secondary efficacy and safety objectives may include the following: percentage of participants achieving HbA1c target (with or without hypoglycemia); change in fasting glucose; rate of hypoglycemia events during the treatment period; change in body weight; development of anti-drug antibodies; and CGM derived endpoints like time in target, above, or below the target range

Study participants and their caregivers, if applicable, will be trained about the signs and symptoms of hyperglycemia and hypoglycemia and how to perform glucose monitoring as per protocol instructions. They may also check their glucose levels as frequently as needed and will be instructed to contact the investigative sites in the event of severe, persistent hyperglycemia or severe hypoglycemia between study visits. Appropriate information for each episode of hypoglycemia will be collected in study diaries.

Self-monitoring of blood glucose or continuous glucose monitoring will be utilized (using a device approved for this purpose) to inform dose adjustments and monitor hyperglycemia and hypoglycemia. Participants who develop severe, persistent hyperglycemia based on specific pre-defined thresholds will receive an additional glucose-lowering intervention (or rescue therapy). Additionally, in cases where patients fulfill the definition of increased risk of hypoglycemia, instructions will be provided to first decrease the dose of BIF per the respective dosing algorithm and then, if necessary, discontinue the study drug. Detailed instructions on how to manage hyperglycemia and hypoglycemia will be provided to investigative sites and study participants. Safety of study participants will be closely monitored throughout the entire study period, including the safety follow up.

In one Phase 3 study, the starting dose for insulin naive participants will be derived from FG and BW, similar to the approach described above in the insulin-naive phase 2 study. An example of such an approach is described below in Table 49:

**Table 49.**

| **Median FG value from 3 days (mg/DL)** | **Body Weight (KG)** | | | |
|---|---|---|---|---|
| | **≤80** | **81-100** | **101-120** | **>120** |
| 100-140 | 120 I.U. | 200 I.U. | 240 I.U. | 280 I.U. |
| 141-180 | 250 I.U. | 280 I.U. | 420 I.U. | 490 I.U. |
| 181-220 | 370 I.U. | 420 I.U. | 560 I.U. | 560 I.U. |
| >220 | 490 I.U. | 560 I.U. | 630 I.U. | 700 I.U. |

For participants who were previously being treated on basal insulins, the starting dose will be determined based on prior basal insulin dose and FG data.

Dose adjustments will be determined based on FG in the previous week. An example set of guidelines for dose adjustments is set forth in Table 50 below:

**Table 50.**

| **Median FPG value from 3 days (mg/DL)** | **Dose adjustment based on previous weeks insulin dose (I.U.)** | | |
|---|---|---|---|
| | **Week 2** | **Week 3** | **Week 4 and subsequent** |
| ≤80 | -70 | -70 | -14 |
| 81-100 | -42 | -28 | No change |
| 101-140 | No change | No change | 14 |
| 141-180 | 120 | 70 | 28 |
| >180 | 210 | 120 | 42 |

In addition to the FG-based guidelines for dose adjustments described above, dose decreases will be implemented based on the occurrence of any of the following instances of hypoglycemia: multiple episodes of recorded hypoglycemia with SMBG <70 mg/dL; severe hypoglycemia (requiring assistance); and/or documented hypoglycemia ≤54 mg/dL in the preceding week. In addition, the dose may not be increased if any SMBG reading was documented at <70 mg/dL at any time in the preceding week.

An additional study is designed to test dosing regimens designed to provide simpler dosing guidelines while still achieving desired glycemic targets, albeit potentially more gradually, and without increasing hypoglycemia risk.

The first dose administered in these studies is differs for insulin-naive participants compared to those previously treated with daily basal insulin. The starting dose for insulin-naive participants is about 70 I.U. Participants previously treated with basal insulin will receive a loading dose based on a 1-to-1 unit conversion from their prior daily insulin dose to a weekly dose times the factor of 3. For example, participants taking 30 I.U. of daily glargine will start with a BIF loading dose of 630 I.U. (30 I.U. × 7 days × 3). This loading dose strategy is designed to achieve an efficacious exposure to reduce transient hyperglycemia during this transition period.

Subsequent weekly dose adjustments for all participants will be based on FG values as shown in Table 51 below:

**Table 51.**

| **FG Value (mg/dL)** | **Dose Adjustment (I.U.)** |
|---|---|
| <54 | -28 |
| 55-70 | -14 |
| 71-100 | No Change |
| 101-125 | 14 |
| >125 | 28 |

In addition, dose decreases will be implemented based on the occurrence of any of the following instances of hypoglycemia: multiple episodes of recorded hypoglycemia with SMBG <70 mg/dL; severe hypoglycemia (requiring assistance); and/or documented hypoglycemia ≤54 mg/dL in the preceding week. In addition, the dose may not be increased if any SMBG reading was documented at <70 mg/dL at any time in the preceding week.

### Sequences

### SEQ ID NO:1

         10         20         30         40         50         60
FVNQHLCGSHLVEALELVCGERGFHYGGGGGGSGGGGGIVEQCCTSTCSLDQLENYCGGG
         70         80         90        100        110        120
GGQGGGGQGGGGQGGGGGECPPCPAPPVAGPSVFLFPPKPKDTLMISRTPEVTCVVVDVS
        130        140        150        160        170        180
HEDPEVQFNWYVDGVEVHNAKTKPREEQFNSTFRVVSVLTVVHQDWLNGKEYKCKVSNKG
        190        200        210        220        230        240
LPAPIEKTISKTKGQPREPQVYTLPPSREEMTKNQVSLTCLVKGFYPSDIAVEWESNGQP
        250        260        270        280        290
ENNYKTTPPMLDSDGSFFLYSKLTVDKSRWQQGNVFSCSVMHEALHNHYTQKSLSLSPG

## Claims

1. Insulin efsitora alfa for use in the treatment of diabetes, wherein the treatment comprises providing glycemic control by:
a) administering to an insulin naive subject an initial dose of weekly insulin efsitora alfa that is a 300 U loading dose; and
b) administering to said subject one or more weekly maintenance doses once-weekly starting one week after administration of the initial dose.

2. Insulin efsitora alfa for use according to claim 1 wherein each weekly maintenance dose is selected according to the following criteria:
a) if the previous dose was the loading dose, the weekly maintenance dose is 100 U; or
b) if the previous dose was not the loading dose, the weekly maintenance dose is equal to the previous weekly maintenance dose adjusted, if necessary, according to the following criteria:
i) decreased by 20 units if the subject's median fasting glucose during the previous week was <80 mg/dL;
ii) equal to the previous dose if the subject's median fasting glucose during the previous week was 80-120 mg/dL;
iii) increased by 20 units if the subject's median fasting glucose during the previous week was 121-140 mg/dL; or
iv) increased by 40 units if the subject's median fasting glucose was >140 mg/dL.

3. Insulin efsitora alfa for use according to any one of claims 1 or 2, wherein the weekly maintenance dose is not increased if the subject had a blood glucose episode < 70 mg/dL in the previous week.

4. Insulin efsitora alfa for use according to any one of claims 1 to 3 wherein the weekly maintenance dose is decreased by 40 units if, in the previous week, the subject had any of:
a) ≥ 3 blood glucose episodes ≤ 70 mg/dL;
b) ≥ 1 nocturnal blood glucose episode ≤ 70 mg/dL;
c) ≥ 1 blood glucose episode ≤ 54 mg/dL; or
d) any episode of severe hypoglycemia.

5. Insulin efsitora alfa for use according to any one of claims 1 to 4 wherein the weekly maintenance dose is not increased if the subject had one or more incidents of hypoglycemia believed to be attributed to insulin efsitora alfa.

6. Insulin efsitora alfa for use according to any one of claims 1 to 5, wherein the weekly maintenance dose is not increased if the subject had one or more incidents of nocturnal hypoglycemia.

7. Insulin efsitora alfa for use according to any one of claims 1 to 5, wherein the weekly maintenance dose is not increased if the subject had one or more incidents of severe hypoglycemia.

8. Insulin efsitora alfa for use according to any one of claims 1 to 7 wherein the need for any weekly maintenance dose adjustments is determined: every week for the first 12 weeks after initiation of treatment with insulin efsitora alfa; and every 4 weeks thereafter.

9. Insulin efsitora alfa for use according to any one of claims 1 to 8 wherein the treatment comprises improving glycemic control in the patient.

## Patentansprüche

1. Insulin efsitora alfa zur Verwendung bei der Behandlung von Diabetes, wobei die Behandlung ein Bereitstellen glykämischer Kontrolle umfasst durch:
a) Verabreichen einer Anfangsdosis von wöchentlichem Insulin efsitora alfa, die eine 300-U-Ladungsdosis ist, an ein insulinnaives Subjekt; und
b) Verabreichen einer oder mehrerer wöchentlicher Erhaltungsdosen an das Subjekt einmal wöchentlich, beginnend eine Woche nach Verabreichung der Anfangsdosis.

2. Insulin efsitora alfa zur Verwendung nach Anspruch 1, wobei jede wöchentliche Erhaltungsdosis gemäß den folgenden Kriterien ausgewählt ist:
a) falls die vorherige Dosis die Ladungsdosis war, die wöchentliche Erhaltungsdosis 100 U beträgt; oder
b) falls die vorherige Dosis nicht die Ladungsdosis war, die wöchentliche Erhaltungsdosis gleich der vorherigen wöchentlichen Erhaltungsdosis ist, angepasst, falls erforderlich, gemäß den folgenden Kriterien:
i) gesenkt um 20 Einheiten, falls die mediane Nüchternglukose des Subjekts während der vorherigen Woche <80 mg/dl betrug;
ii) gleich der vorherigen Dosis, falls die mediane Nüchternglukose des Subjekts während der vorherigen Woche 80-120 mg/dl betrug;
iii) erhöht um 20 Einheiten, falls die mediane Nüchternglukose des Subjekts während der vorherigen Woche 121-140 mg/dl betrug; oder
iv) erhöht um 40 Einheiten, falls die mediane Nüchternglukose des Subjekts >140 mg/dl betrug.

3. Insulin efsitora alfa zur Verwendung nach einem der Ansprüche 1 oder 2, wobei die wöchentliche Erhaltungsdosis nicht erhöht wird, falls das Subjekt in der vorherigen Woche eine Blutglukoseepisode < 70 mg/dl aufwies.

4. Insulin efsitora alfa zur Verwendung nach einem der Ansprüche 1 bis 3, wobei die wöchentliche Erhaltungsdosis um 40 Einheiten gesenkt wird, falls das Subjekt in der vorherigen Woche jegliches aufwies von:
a) ≥ 3 Blutglukoseepisoden ≤ 70 mg/dl;
b) ≥ 1 nächtliche Blutglukoseepisode ≤ 70 mg/dl;
c) ≥ 1 Blutglukoseepisode ≤ 54 mg/dl; oder
d) jeglicher Episode schwerer Hypoglykämie.

5. Insulin efsitora alfa zur Verwendung nach einem der Ansprüche 1 bis 4, wobei die wöchentliche Erhaltungsdosis nicht erhöht wird, falls das Subjekt ein oder mehrere Ereignisse von Hypoglykämie, von denen angenommen wird, dass sie auf Insulin efsitora alfa zurückzuführen sind, aufwies.

6. Insulin efsitora alfa zur Verwendung nach einem der Ansprüche 1 bis 5, wobei die wöchentliche Erhaltungsdosis nicht erhöht wird, falls das Subjekt ein oder mehrere Ereignisse nächtlicher Hypoglykämie aufwies.

7. Insulin efsitora alfa zur Verwendung nach einem der Ansprüche 1 bis 5, wobei die wöchentliche Erhaltungsdosis nicht erhöht wird, falls das Subjekt ein oder mehrere Ereignisse schwerer Hypoglykämie aufwies.

8. Insulin efsitora alfa zur Verwendung nach einem der Ansprüche 1 bis 7, wobei die Notwendigkeit jeglicher wöchentlicher Erhaltungsdosisanpassungen bestimmt wird: jede Woche für die ersten 12 Wochen nach Anfang der Behandlung mit Insulin efsitora alfa; und danach alle 4 Wochen.

9. Insulin efsitora alfa zur Verwendung nach einem der Ansprüche 1 bis 8, wobei die Behandlung ein Verbessern glykämischer Kontrolle bei dem Patienten umfasst.

## Revendications

1. Insuline efsitora alfa pour utilisation dans le traitement du diabète, dans laquelle le traitement comprend la fourniture d'une régulation glycémique par :
a) l'administration à un sujet n'ayant jamais reçu d'insuline d'une dose initiale hebdomadaire d'insuline efsitora alfa qui est une dose d'attaque de 300 U ; et
b) l'administration audit sujet d'une ou plusieurs doses d'entretien hebdomadaires une fois par semaine en commençant une semaine après administration de la dose initiale.

2. Insuline efsitora alfa pour utilisation selon la revendication 1 dans laquelle chaque dose d'entretien hebdomadaire est sélectionnée selon les critères suivants :
a) si la dose précédente était la dose d'attaque, la dose d'entretien hebdomadaire est de 100 U ; ou
b) si la dose précédente n'était pas la dose d'attaque, la dose d'entretien hebdomadaire est égale à la dose d'entretien hebdomadaire précédente ajustée, si nécessaire, selon les critères suivants :
i) diminuée de 20 unités si le glucose à jeun médian du sujet pendant la semaine précédente était <80 mg/dL ;
ii) égale à la dose précédente si le glucose à jeun médian du sujet pendant la semaine précédente était de 80 à 120 mg/dL ;
iii) augmentée de 20 unités si le glucose à jeun médian du sujet pendant la semaine précédente était de 121 à 140 mg/dL ; ou
iv) augmentée de 40 unités si le glucose à jeun médian du sujet était >140 mg/dL.

3. Insuline efsitora alfa pour utilisation selon l'une quelconque des revendications 1 ou 2, dans laquelle la dose d'entretien hebdomadaire n'est pas augmentée si le sujet a eu un épisode de glycémie < 70 mg/dL dans la semaine précédente.

4. Insuline efsitora alfa pour utilisation selon l'une quelconque des revendications 1 à 3 dans laquelle la dose d'entretien hebdomadaire est diminuée de 40 unités si, dans la semaine précédente, le sujet a eu l'un quelconque parmi :
a) ≥ 3 épisodes de glycémie ≤ 70 mg/dL ;
b) ≥ 1 épisode de glycémie nocturne ≤ 70 mg/dL ;
c) ≥ 1 épisode de glycémie ≤ 54 mg/dL ; ou
d) l'un quelconque épisode d'hypoglycémie sévère.

5. Insuline efsitora alfa pour utilisation selon l'une quelconque des revendications 1 à 4 dans laquelle la dose d'entretien hebdomadaire n'est pas augmentée si le sujet a eu un ou plusieurs incidents d'hypoglycémie que l'on pense être attribués à l'insuline efsitora alfa.

6. Insuline efsitora alfa pour utilisation selon l'une quelconque des revendications 1 à 5, dans laquelle la dose d'entretien hebdomadaire n'est pas augmentée si le sujet a eu un ou plusieurs incidents d'hypoglycémie nocturne.

7. Insuline efsitora alfa pour utilisation selon l'une quelconque des revendications 1 à 5, dans laquelle la dose d'entretien hebdomadaire n'est pas augmentée si le sujet a eu un ou plusieurs incidents d'hypoglycémie sévère.

8. Insuline efsitora alfa pour utilisation selon l'une quelconque des revendications 1 à 7 dans laquelle la nécessité de quelconques ajustements de dose d'entretien hebdomadaire est déterminée : chaque semaine pendant les 12 premières semaines après initiation du traitement avec l'insuline efsitora alfa ; et toutes les 4 semaines par la suite.

9. Insuline efsitora alfa pour utilisation selon l'une quelconque des revendications 1 à 8 dans laquelle le traitement comprend l'amélioration de régulation glycémique chez le patient.
